# EUROPEAN PATENT APPLICATION

(11) **EP 3 790 021 A1**
(43) Date of publication of application: **10.03.2021**
(21) Application number: 19196226.5
(22) Date of filing: 09.09.2019
(51) Int. Cl.: G16H 40/40, A61M 1/36

(54) **METHOD OF DEACTIVATION, SYNCHRONIZATION AND DETECTING PROXIMITY, MEDICAL COMPUTING UNIT AND MEDICAL DEVICE CONFIGURED FOR SUCH METHODS**

(71) Applicant: Fresenius Medical Care Deutschland GmbH, 61352 Bad Homburg (DE)
(72) Inventor: Stahl, Thomas, 97839 Esselbach (DE); Thorwarth, Michael, 60486 Frankfurt am Main (DE); Syfonios, Andreas, 97493 Bergrheinfeld (DE); Millán-Gatante, Maria, 61352 Bad Homburg (DE); Eitel. Stefan, 97289 Thüngen (DE); Schreiber, Alexander, 36142 Tann (Rhön) (DE)
(74) Representative: Bobbert & Partner Patentanwälte PartmbB

(57) **Abstract**

The invention comprises a method of deactivation of a medical computing unit (100), wherein the medical computing unit (100) is deactivated if a predefined condition is met and a method of data synchronization in which a first medical computing unit (101) reads data from a medical device (1) and the first medical computing unit (101) transmits the read data to a second medical computing unit (102). Furthermore, the invention comprises a method of detecting proximity between a medical device (1) and a medical computing unit (100) with a wireless data transfer unit comprising the step of detecting by the medical computing unit (100) and/or the medical device (1) if the medical computing unit (100) is located in close proximity to the medical device (1). In addition, the invention comprises a medical computing unit (100), a medical device (1) and a system of medical computing unit (100) and medical device (1).

## Description

The present invention relates to a method of deactivation, synchronization and detecting proximity. Furthermore, the present invention relates to a medical computing unit and a medical device. Additionally, the invention comprises a digital storage medium, a computer program product and a computer program.

When using medical computing units, unauthorized access is undesirable in many circumstances. When several medical computing units are used by medical staff to check treatment data, it may be desirable to have the treatment data synchronized across all medical computing units. Additionally, if medical computing units contain wireless data transfer units, it must be ascertained that the medical computing units remain at a safe distance from the medical device to avoid harmful interference.

It is an object of the present invention to suggest improved methods for deactivation, synchronization and detecting proximity. In addition, improved medical computing units and medical devices configured for such methods are an object of the invention. An improved medical device with a protective element is another object of the invention. Furthermore, it is an object to propose an improved digital storage medium, computer program product and computer program for such a medical computing unit and medical device.

The object according to the present invention is achieved by the method of deactivation having the features of claim 1, by the method of data synchronization having the features of claim 6 and by the method of detecting proximity comprising the features of claim 9. The object is achieved by the medical computing unit according to claim 12, by the medical device according to claim 13 and 14 as well as by the system of claim 17. It is further achieved by the digital storage medium having the features of claim 18, the computer program product having the features of claim 19 as well as the computer program having the features of claim 20.

In all of the aforementioned and following statements, the use of the expression "may be" or "may have", etc., is to be understood synonymously with "preferably is" or "preferably has", etc., respectively, and is intended to illustrate an embodiment according to the present invention.

Whenever numerical words are mentioned herein, the person skilled in the art shall understand them as indications of numerical lower limits. Hence, unless this leads to a contradiction evident to the person skilled in the art, the person skilled in the art shall comprehend for example "one" as encompassing "at least one". This understanding is also equally encompassed by the present invention as the interpretation that a numerical word, for example, "one" may alternatively mean "exactly one", wherever this is technically possible in the view of the person skilled in the art. Both meanings are encompassed by the present invention and apply herein to all used numerical words.

Whenever the terms "programmed" or "configured" are mentioned herein, these terms are interchangeable.

The information "top" and "bottom" are to be understood as spatial information referring to the situation or orientation of the respective element during intended use.

The invention comprises a method of deactivation of a medical computing unit. The medical computing unit is suitable, in particular configured, for processing patient data. Patient data may be or include, e.g., personal details, such as name and address of the patient, or measurement data, such as blood pressure or glucose level, or data on diagnosis and treatments of the patient, such as the patient having a particular condition. The medical computing unit is deactivated if a predefined condition is met. If the medical computing unit contains sensitive data, it is important that unauthorized access be prevented. Depending on the predefined condition, such unauthorized access may be blocked.

The method of data synchronization according to the invention comprises the following steps:
- a first medical computing unit reads data from a medical device
- the first medical computing unit transmits the read data to a second medical computing unit.

The medical device may be a blood treatment apparatus. The blood treatment apparatus may be a dialysis machine.

The first medical computing unit may be the same type of device as the second medical computing unit. Alternatively, the two medical computing units may be of different type. The method comprises the embodiment in which the first medical computing unit transmits the data not just to the second medical computing unit, but also to a third, fourth, fifth medical computing unit etc. of identical or different type.

The medical computing unit may be, e.g., a mobile device, such as a tablet computer, a smartphone or a laptop.

Furthermore, the invention comprises a method of detecting proximity between the medical device and the medical computing unit.

In the method of detecting proximity, the medical computing unit comprises a wireless data transfer unit, such as a Wi-Fi or Bluetooth module. In the method, the medical computing unit and/or the medical device detects whether the medical computing unit is located in close proximity to the medical device.

The medical computing unit according to claim 12 is configured to execute the method of deactivation, the method of synchronization and/or the method of detecting proximity according to the invention.

The medical device is configured to execute the method of deactivation in some embodiments, the method of synchronization and/or the method of detecting proximity according to the invention.

The invention comprises a medical device with a protective element, which protects against the emitted electromagnetic radiation of a medical computing unit.

The electromagnetic radiation emitted by the medical computing unit with a wireless data transfer unit may cause malfunction of the medical device if the medical computing unit is in close proximity to the medical device. Advantageously, the protective element may reduce electromagnetic radiation from the medical computing unit reaching the medical device.

The system of the invention comprises at least one mobile medical computing unit according to claim 12 and at least one medical device according to claim 13 to 16. The described medical devices and medical computing units work in conjunction and can take their full effect working together in the system.

According to the invention, a digital, in particular nonvolatile, storage medium, in particular in the form of a machine readable carrier, in particular in the form of a floppy disk, CD, DVD or an EPROM, in particular with electronically or optically readable control signals, is configured to interact with the medical device and/or the medical computing unit, such that the machine-induced steps of the methods according to the invention are prompted.

The computer program product according to the invention comprises a program code saved on a machine-readable carrier for prompting the machine-induced steps of the methods according to the invention when the computer program product runs on the medical device and/or the medical computing unit.

A computer program according to the invention comprises a program code for prompting the machine-induced steps of the methods according to the invention, when the computer program runs on the medical device and/or the medical computing unit.

In locations with suboptimal and unreliable IT infrastructure where medical devices, such as dialysis machines, are used, there is a need for resilient setups. In such locations, dialysis machines without data interfaces are often used due to lower cost.

In dialysis wards or clinics in such locations, many different dialysis treatments are carried out concurrently. During the treatment, a large amount of data is accrued (treatment data, machine data and other data), which must be recorded by medical personnel for documentation purposes. In a dialysis machine without data interfaces, data may be read from a display of the dialysis machine and written down manually by staff, which is a source of error and puts a strain on staff productivity.

For recording of data, notepaper is sometimes used, which is stuck to clipboards on the dialysis machines. The dialysis nurse writes relevant data on the paper during the treatment. Then the nurse adds their signature to identify themselves as the treating person. After the treatment, the paper is kept or the relevant information is stored electronically in a database, on a computer or via scan.

This process is cumbersome, laborious and prone to error. The time used on such tasks is taken away from time spent caring for the patients. It is one important aspect of the invention to simplify documentation tasks and make them less prone to error.

To this end, it is proposed to display one- or two-dimensional graphical representations on a display of the medical device, wherein the data to be documented is contained in the graphical representations. Such data could be, e.g., physiological data of the patient, patient identity information, treatment information, status messages of the medical device, such as the status of self-tests carried out by the medical device, levels of fluid supply at the medical device, etc.

In many cases, older medical devices, such as older dialysis machines, comprise a display, which permits showing of such graphical representations in a suitable resolution. In other cases, older dialysis machines do not contain a suitable display. Therefore, a display unit for displaying graphical representations (such as bar codes, QR codes and/or flicker codes) is provided for such medical devices, which do not comprise a suitable display, but which comprise, e.g., a data interface (e.g., a serial interface). The necessary data transfer from the dialysis machine to the display unit may be implemented by utilising the existing data interface.

The data displayed as a graphical representation is readable by the medical computing unit and can be decoded by the medical computing unit.

In some embodiments, the graphical representation is recorded by the medical computing unit and then transmitted as an image file to a second device (e.g., a server or a medical computing unit of the same type) and is only decoded on the second device.

The medical device is preferably programmed such that certain data (e.g., information on the treatment, the patient or the medical device) is coded in the graphical representation, which is then automatically displayed on the display of the medical device.

This preferably occurs in fixed time intervals. The graphical representation shown is preferably linked to a certain time, e.g., the time of creation of the graphical representation. This time may be contained in the graphical representation or the medical computing unit reading the graphical representation may link the time of reading with the graphical representation read at that time.

If the time of its creation is contained within the graphical representation, then the medical computing unit reading the graphical representation can often deduce whether a graphical representation has not yet been read. The detection of a missing graphical representation, i.e., a graphical representation, which has not been read yet, may occur if graphical representations are created in fixed time intervals and the time between two graphical representations read is longer than the time interval. For example, if the time between the latest graphical representation read and the graphical representation read immediately before is longer than the fixed interval then a missing graphical representation is detected.

In another embodiment, missing graphical representations may be detected by a consecutive numbering of graphical representations. If the numbering of the graphical representations read is not consecutive, then there is at least one graphical representation missing. In this way, the periodic reading of graphical representations can be evaluated.

In general, it is possible to reprogram older medical devices without a data interface to transmit data to the external display unit. This may be implemented by controlling an LED of the medical device to transmit to the external display unit, e.g., via a serial blink code. In conjunction with a corresponding optical sensor, the blinking may be recorded and processed such that the external display unit may display the graphical representation containing the transferred data. Due to the serial optical data transfer, this process may take longer than alternative methods. This should, however, not be problematic due to the long reading intervals.

In some embodiments, the medical computing unit in conjunction with the medical device comprises a complete administration application for clinical dialysis, which preferably implements an entire dialysis workflow.

For data exchange, a local wireless network may be built, which connects the medical computing units, which are used by medical personnel, with a central device (e.g., a local server or PC), which transfers data to a storage provider, such as a central server or a cloud storage provider.

In certain situations, e.g., in some countries with unreliable electricity supply, the permanent availability of the local network may not be guaranteed. However, for the documentation of treatment data it is vital that the data recorded by the medical computing units be unambiguously and completely transferred. To this end, the medical computing units preferably possess a data storage for storing the recorded data, wherein the data sets are linked to the time point when the data was recorded.

Furthermore, the medical computing units preferably comprise the ability to perform a network availability check. The network availability check verifies whether there is an existing connection to the local central device (e.g., via periodic handshaking). If no connection is present, then the medical computing unit cannot transfer data to the local central device and may instead store the data in a buffer storage.

It is preferable if all medical computing units, which are used in the ward by different persons, contain the same data stock to avoid duplicate transmission of identical data. To this end, the data stock may be synchronised regularly, e.g., after a set period of time and/or whenever new data arrives at one or more medical computing units and/or at the central device.

The synchronization may be initiated by the central device, if a connection with some or all of the medical computing units exists. When no network connection is present, another possibility of synchronising data between participating medical computing units is fog computing. In this case, a network connection to the central device is not necessary. Instead, the participating medical computing units may synchronise their data via a peer-to-peer connection. Any wireless interface may be suitable for such synchronization, e.g., Wi-Fi peer-to-peer, Bluetooth or infrared interfaces.

To avoid duplicate entries during data synchronization, in some embodiments, the medical computing units may be able to differentiate between data acquired from a medical device (original information) and data received by another medical computing unit (copied information). In some embodiments, for data synchronization, only original information is exchanged. In this way, one data set is only distributed once.

In another embodiment, each medical computing unit knows how many other medical computing units exist and are online (i.e., currently used) in the entire setup. This may, e.g., be announced by the central device, to which the medical computing units may register.

Alternatively, the medical computing units, which are used, send a message to all the other medical computing units via fog computing. After data synchronization, a response message follows (after a certain period of time), which contains information on how many and which medical computing units have carried out data synchronization. Each medical computing unit, which receives data from another medical computing unit, may confirm the receipt of the data to the transmitting medical computing unit.

In some embodiments, when response messages lead to the conclusion that not all medical computing units have carried out data synchronization, copied information is transmitted by any or by certain medical computing units to the medical computing unit, which has not received the information previously. This is particularly useful if the medical computing unit, which has yet to receive the information, is out of range from the medical computing unit containing the original information.

In some embodiments, the copied information is sent consecutively from each medical computing unit until the medical computing unit, which has yet to receive the information, sends an acknowledgement of receipt. If a certain medical computing unit sends copy information and does not receive an acknowledgement of receipt, e.g., the sending medical computing unit sends a message to another medical computing unit or to all medical computing units, which leads to another medical computing unit making a data transmission attempt. The order in which the medical computing units send copied information may be set in advance, e.g., on the basis of a network identification, MAC address, IP address etc.

If the transmission of copied information remains unsuccessful after every medical computing unit has attempted to send the information to the medical computing unit, which has yet to receive the information, this may be documented. In this case, the medical computing unit, which has yet to receive the information, may be out of range, out of charge or defective. The information that the medical computing unit cannot be reached may be used to deactivate or block the medical computing unit for further use until its proper use and correct data synchronization has been ensured.

In another embodiment, medical computing units are safeguarded against improper or unauthorized use. An improper use may, e.g., be the use of the medical computing unit outside the clinical setting. An unauthorized use comprises, e.g., the use of the medical computing unit outside the intended clinical location. In some embodiments, a satellite navigation module (e.g., GPS, GLONASS, Galileo or Baidou module) detects the location of the medical computing unit and the medical computing unit is programmed such that it only operates in the intended location.

Preferably, the medical computing unit is set up to prevent unauthorized use. To this end, the user (e.g., the nurse) may carry a barcode or a QR code (batch), which uniquely identifies the user. In order to unlock the medical computing unit, the code may be read by the medical computing unit.

In order to prevent that the medical computing unit be used by unauthorized persons, even if the nurse did not lock the medical computing unit after use, the medical computing unit may lock automatically. This may occur when the medical computing unit has not been used (e.g., no interaction with the medical computing unit, no data entry and/or no touching of the screen) for a certain period of time. In some embodiments, an internal IMU (inertial measurement unit) or an orientation sensor may be used to detect phases in which the medical computing unit is not used, e.g., when the nurse puts the medical computing unit aside.

In an alternative embodiment, the medical computing unit is locked depending on its location. If the medical computing unit is not in the treatment area, but in another room (e.g., to charge its battery), the medical computing unit can be locked automatically. To detect the location of the medical computing unit, e.g., Wi-Fi signal strength may be used. Alternatively or additionally, the medical computing unit may lock when it is charging.

Further possibilities for unlocking and identifying the user are: Recognizing fingerprints, facial recognition and/or iris scans. The medical computing units are set up such that only certain users may unlock them. The information on the identity of the user may be used for the documentation of treatment.

As described above, the reading of treatment data encoded in QR codes by medical computing units enables a secure and simple documentation of treatment. Additionally, a software is provided, which allows the further administration of the dialysis ward.

In some embodiments, the software assigns specific patients to specific medical personnel. In this, the software accounts for the documented and/or current health status of the respective patients. In this way, particularly unstable patients or patients with specific needs may be assigned to certain nurses, e.g., nurses which are particularly experienced or which have obtained specialist training. Whether a patient is considered to be unstable may be determined according to their documented physiological data and/or according to their current state of health. The patient may be weighed to detect their pre-dialysis weight, which is relevant for their ultrafiltration volume. An unusual deviation from this wet weight could be the deciding factor to assign a patient to a specific nurse. Such an assignment may also be made based on physiological data recorded during dialysis (blood pressure, pulse, etc.) or according to the patient's personal preference.

Such preference may also lead to a certain treatment space, which is agreeable to the patient, or which is adjacent to another patient.

In another embodiment, the software comprises a messaging or chat module, which allows the medical personnel to communicate with each other. This may occur symmetrically in real time across several medical computing units (e.g., as an online chat or an audio/video communication system). In another embodiment, the communication occurs asymmetrically, i.e., by leaving a message (as a text, audio or video message) on the medical computing unit, which may be directed to one or more certified users of the system. This message may be read and replied to later by the addressee. The asymmetric communication also functions with just one medical computing unit, which is used consecutively by different certified users.

In another embodiment, the software comprises a module, which immediately adapts the scheduling (i.e., the planning of treatments, in particular the assignment of treatment periods to patients in a dialysis ward) due to a no show of a patient to enable optimal use of resources. In this context, an ad hoc scheduling of patients without appointments (emergencies, holiday patients, etc.) can be carried out.

In another embodiment, the software comprises a prescription module. Such a module comprises the recording of treatment data (e.g., dialysis duration, ultrafiltration rate, dialysis solution, type of therapy such as hemofiltration, hemodiafiltration, used disposables and solutions, etc.) for a patient, e.g., via a database, which contains this data (e.g., a hospital database) or via data exchange with the treating physician and an assignment to a treatment space, i.e., a specific dialysis machine. The nurse may, e.g., scan the QR code displayed at or attached to the dialysis machine, which leads to an assignment via scheduling or via a scanned patient identification code (QR code armband) and the prescription is displayed. In an embodiment, the system comprises a bidirectional connection between tablet and dialysis machine, which allows the automatic transmission of the prescription.

In another embodiment, the software comprises a report module, which records the collected treatment data as described above and sends it in fixed but freely configurable format to an instance (e.g., a server or an accounting center).

In this context, the software may provide a billing module, which enables the calculation and billing of medical services due to the report. In another embodiment, the software may be able to receive payments from the patient via a credit card or other payment systems.

In some embodiments, the medical computing units may comprise a wireless data transfer unit, such as a Wi-Fi module. When such a medical computing unit is placed on top of the medical device, the resulting electromagnetic field strength entering the dialysis machine may be beyond safety limits of the medical device. One solution is to provide the medical device with a spacer, which keeps the wireless data transfer unit of the medical computing unit far enough away from the relevant locations inside the medical device. Alternatively, the medical device may be constructed such that a medical computing unit cannot be placed on top of it, e.g., by designing a surface, which does not allow the placement of the medical computing unit.

In another alternative, the medical computing unit and/or the medical device detects the placement of the medical computing unit on the medical device, which leads to the deactivation of the wireless data transfer unit or to the reduction of its signal power. Whether the medical computing unit has been placed on the medical device may be observed, e.g., by a camera in the medical computing unit or in the medical device. Image recognition may lead to the detection of such placement. In the case, in which the camera is in the medical computing unit, the medical computing unit may switch off the wireless data transfer unit or reduce its power. Alternatively, the medical computing unit may issue a visual and/or an acoustic warning.

If the camera is in the medical device, the medical device may send a signal, e.g., a wireless signal, to the medical computing unit, which may lead to the medical computing unit switching off its wireless data transfer unit or to the reduction of its power and/or to a visual and/or acoustic warning.

Advantageous developments of the present invention are each subject matter of the dependent claims and embodiments.

Whenever an embodiment is mentioned herein, it is to be understood as an exemplary embodiment according to the present invention.

Embodiments according to the present invention may comprise one or several of the features mentioned herein in any feasible combination.

In the method of deactivation of the medical computing unit, some embodiments implement one or more of the following predefined conditions, which trigger deactivation. In some embodiments, deactivation will be triggered if only one of a group of conditions is met, in other embodiments, all conditions of a group must be met to trigger deactivation.

In some embodiments, deactivation of the medical computing unit is triggered, if a touch screen of the medical computing unit has not been touched for a specific period of time. Such a period of time can, e.g., be settable in a range between 15 s and 120 s. Causing deactivation after a set period of time increases device safety. In a clinical setting, tasks are often interrupted by sudden urgent matters occurring. When the medical computing unit is then set aside, unauthorized access could occur. The deactivation after a set period of time reduces this risk.

In some embodiments, an MPU, such as an accelerometer and/or magnetometer, is contained in the medical computing unit. If the MPU does not detect any movement of the device for a set period of time, the medical computing unit may be deactivated. If no movement is detected, it is likely that the medical computing unit has been put down and is not with the user anymore. It is then safer to deactivate the medical computing unit.

In some embodiments, the medical computing unit is deactivated, if it has not been used for a set period of time. The use may be detected in a number of ways, e.g., data entry or use of the camera. If no use has been detected, it increases device safety to deactivate the medical computing unit.

In some embodiments, the medical computing unit is deactivated, if the medical computing unit is placed inside or outside a predefined area. An area may be defined in which the medical computing unit may be used exclusively. When the medical computing unit leaves this area, it will be deactivated. In some cases, it may be deactivated temporarily. In other cases, the medical computing unit may be deactivated permanently. Such a deactivation prevents device theft and increases data safety. If an irreversible deactivation occurs, when the medical computing unit is removed from the area, device theft becomes less attractive. Also, the risk of patient data being stolen is reduced by this condition. Alternatively, the deactivation may be triggered by entering a certain area. Such an area could be, e.g., an entrance area of the clinic. As a result, any device passing the entrance area would be deactivated.

For such a deactivation based on location, the medical computing unit must be able to locate itself. In some embodiments, the medical computing unit may comprise a satellite navigation module (e.g., GPS, GLONASS, Galileo or Baidou) for a precise location detection. In many situations, an exact determination of location is not important. Location could be detected by the strength of the clinic Wi-Fi signal. When a Wi-Fi signal is lost, in some embodiments, the medical computing unit is deactivated. Alternatively, special beacons may be installed in the rooms of the clinic, which determine the areas, in which the medical computing units can be used. Any movement outside the range of these beacons would then trigger deactivation.

In some embodiments, connecting the medical computing unit to a charger or running out of battery power causes deactivation. Connecting to a charger indicates that the medical computing unit will not be carried around and can thus not be used for many required tasks. Losing battery power indicates that the medical computing unit has been used for a long time. Charging must occur periodically. If charging is not carried out, the medical computing unit will run out of power. Either way, after a certain period of time, the medical computing unit is deactivated. This prevents a stolen device from being used for extended periods of time. After a temporary deactivation, the medical computing unit may be reactivated. Reactivation may be carried out by entering a numeric or alphanumeric code, by scanning a tag, such as a barcode or QR code, or by scanning biometric features, such as a fingerprint. After certain conditions have occurred, the reactivation may only be carried out by a supervisor or a specialist. Finally, after a permanent deactivation, e.g., as a result of a detected theft, the medical computing unit cannot be reactivated again and is of no use to the offending person.

In some embodiments, the medical computing unit is configured for reading data from at least one medical device. To this end, the medical computing unit may be equipped with a sensor to read data from the medical device.

In some embodiments, the medical computing unit reads data from the medical device optically, i.e., via an optical sensor, such as a camera, a photodiode or a phototransistor. A camera may be used to record graphical representations of data from the medical device. A photodiode or phototransistor may be used to record data transmitted by a serial blink code from an LED of the medical device. The optical transfer of data does not need network infrastructure and may, thus, be more robust than wireless data transfer.

In some embodiments, the medical device is a blood treatment apparatus, in particular a dialysis machine.

In the method of synchronization, the first medical computing unit, which has recorded data from a medical device by reading a graphical representation from the display of the medical device, can transmit data to the second, third, fourth, fifth device etc. In one embodiment, only that medical computing unit, which has obtained the data directly by reading the graphical representation from the medical device, may transmit the data to other medical computing units. Alternatively, medical computing units may pass on data, which they have obtained indirectly, i.e., from other medical computing units. In one embodiment, at first, the data is only transferred by medical computing units, which have obtained it directly from a medical device. If this method fails to deliver all data to all medical computing units, then medical computing units send data obtained indirectly to reach the remaining medical computing units. This embodiment may be desirable, if there is no fixed network infrastructure and the medical computing units communicate with each other via peer-to-peer networks etc.

In some embodiments, the medical computing unit reads data from graphical representations on the displays of medical devices. In one example, the medical device may encode data to be transmitted into a static or dynamic graphical representation. The graphical representation may then be shown on a display of the medical device.

The graphical representation may be, e.g., a 1D or 2D barcode such as a QR code. In some embodiments, the graphical representation is dynamic. The dynamic graphical representation comprises a series of several images, which are shown sequentially, e.g., like a film. The dynamic graphical representation may comprise several 1D or 2D barcodes shown in sequence, e.g., as a loop. In one embodiment, the dynamic graphical representation is a flickercode.

In some embodiments, the method of proximity detection according to the invention uses at least one sensor. The sensor may be located on the medical computing unit and/or on the medical device. In one embodiment, a camera of the medical computing unit detects if the medical device is close, e.g., by image recognition. In some embodiments, the medical device uses a sensor to measure a level of electromagnetic radiation of the medical computing unit to detect proximity.

In some embodiments, if proximity between medical computing unit and medical device has been detected, at least one of the following steps is carried out:
- reducing the power of the wireless data transfer unit of the medical computing unit;
- switching off the wireless data transfer unit of the medical computing unit; and/or
- playing a warning message on the medical computing unit and/or the medical device.

By executing these steps, the electromagnetic radiation received at the medical device may be reduced.

In some embodiments, the medical device comprises a spacer to reduce electromagnetic radiation. The spacer may prevent the medical computing unit from being placed too close to the medical device. An external surface could be mounted to the medical device to prevent the medical computing unit from coming too close.

Alternatively or additionally, the medical device may be designed such that the medical computing unit cannot be stably placed on any surfaces thereof. To this end, any horizontal surfaces on the medical device could be changed into slanted or convex surfaces from which the medical computing unit would slide off. In another embodiment, the surface of the medical computing unit could be designed in a way that it does not allow placement on the medical device, e.g., by having a convex backside.

The present invention is explained using accompanying figures in which identical reference numerals refer to same or similar components. The following applies in the figures:
- **Fig. 1**: shows schematically simplified the medical device and the medical computing unit according to the present invention in an exemplary embodiment;
- **Fig. 2**: shows a workflow involving the methods and devices according to the invention;
- **Fig. 3**: shows the display of the medical device according to the invention with three medical computing units according to the invention;
- **Fig. 4**: shows in a simplified schematic the medical device according to the invention;
- **Fig. 5**: shows the medical device according to the invention with a protective surface;
and
- **Fig. 6 to 36**: show several different screens of the medical computing unit according to the invention.

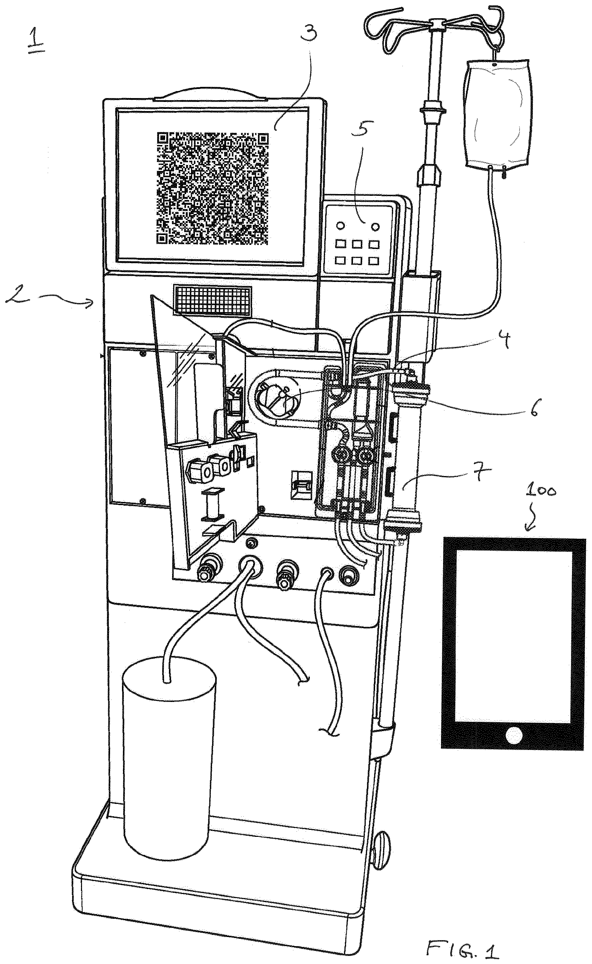

**Fig. 1** shows a medical device 1 implemented as a dialysis machine 2. The medical device 1 comprises a display 3, which serves to present treatment data and graphical representations of such data, e.g., a QR code. The dialysis machine further comprises a blood line set 4 secured to the dialysis machine 2. The dialysis machine 2 comprises a control unit 5, a blood pump 6 and a dialyzer 7 containing a first and a second chamber divided by a membrane. During dialysis, the blood pump 6 pumps blood through a first chamber of a dialyzer 7 connected to the blood line set 4. At the same time, dialysate is pumped through a second chamber of the dialyzer 7. Substances, which have built up in the blood and are to be removed, move across the membrane of the dialyzer 7 from the blood side to the dialysate side. Thus, the blood is cleaned from these substances. **Fig. 1** also shows the medical computing unit 100, which may be used to read the graphical representation from the display 3 of the medical device 1.

In the method of proximity detection according to the invention, a sensor of the medical computing unit 100 or of the medical device 1 detects the relative proximity of the two devices. Such a sensor may be the camera of a medical computing unit 100, which may be implemented as a tablet. Alternatively, the medical device 1 may contain a sensor detecting the emitted electromagnetic radiation of the medical computing unit 100. After the sensor has detected close proximity, a warning is sounded and/or the wireless data transfer unit of the medical computing unit 100 is disabled or its radiated field strength is reduced.

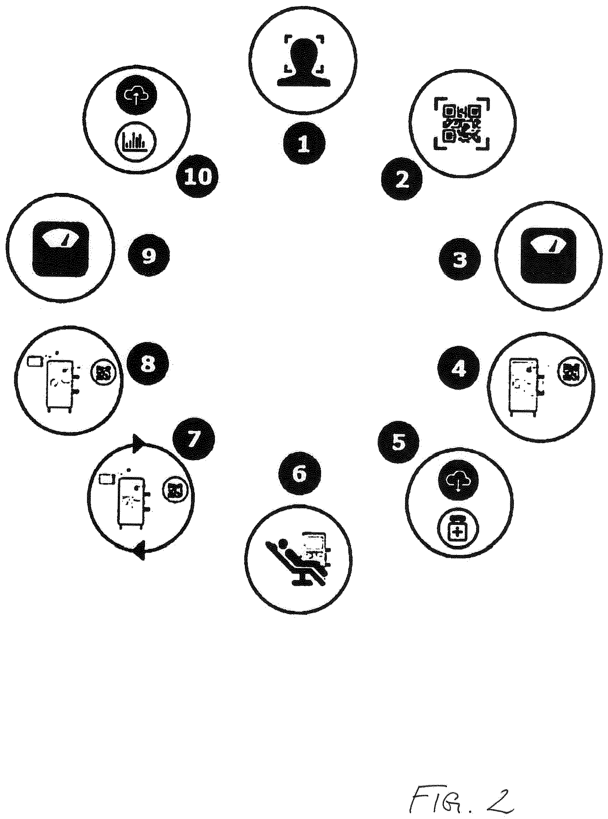

**Fig. 2** shows a workflow involving the methods of the invention. Before the workflow starts, the nurse logs into the medical computing unit 100, such as a tablet, using a PIN code or a personal QR code tag. The tablet shows the nurse how many dialysis machines 2 are already disinfected, tested and ready to use. In addition, the tablet shows a list of patients whom the nurse will treat during their shift. The nurse can add patients to the list. Once a dialysis machine 2 is tested, the nurse can enter the result of the test into the tablet. Alternatively, the dialysis machine 2 shows the result of the test as a graphical representation (e.g., QR code) for the nurse to scan with the tablet. Once the nurse has scanned the graphical representation of a positive test, the dialysis machine 2 appears on the tablet accordingly. If the test is negative and the nurse scans the graphical representation, the corresponding error message appears on the tablet, possibly with suggestions on how to proceed. The nurse may then select a patient from the patient list. The patient list may contain the patient name, the kind of treatment to be performed and the disease status, such as the infectious disease status. The nurse may alternatively enter a patient name manually. After identifying the patient (step 1), the patient may be registered (step 2). Registration may be performed manually at the tablet or by scanning an ID tag of the patient, such as a hospital armband with a barcode. After that, the patient is weighed (step 3) to obtain the pre-treatment weight. The weight is then entered into the tablet. The nurse assigns the patient to a dialysis machine (step 4) and downloads the prescription, i.e., the parameters of the treatment to be performed as prescribed by a doctor (step 5). Subsequently, the treatment is started (step 6). The nurse may track the treatment by scanning graphical representations on the dialysis machine display 3 (step 7). In this way, e.g., blood pressure data may be transferred to the tablet at regular intervals. When the treatment ends, the nurse scans the treatment summary from the dialysis machine 2 (step 8). The patient is then weighed again to measure post-treatment weight (step 9), which is entered into the tablet. Finally, the treatment session is closed on the tablet and the recorded data is transferred from the tablet to another device (step 10). This other device may be a local or central server or a cloud service. The data may also be transferred to one or more other tablets, such that the staff member, who will treat the patient next time, will have access to the data. After the treatment, the tablet can show the nurse, which further steps should be performed, such as wiping of surfaces, machine disinfection and checking for residue. Before, during and/or after the treatment, the nurse may add notes to the patient file on the tablet.

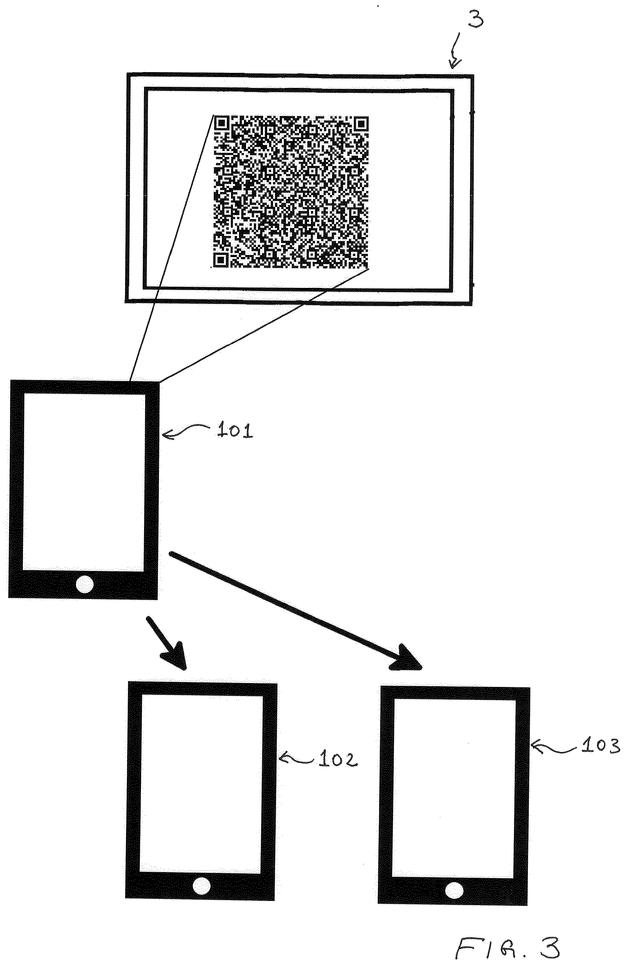

**Fig. 3** shows the display 3 of the medical device 1 according to the invention. The display 3 shows a graphical representation of data (here shown as a QR code), e.g., treatment data from a dialysis treatment. A first medical computing unit 101 uses a camera to take a picture of the graphical representation and decodes the data. The data is then sent via a wireless protocol, such as Wi-Fi or Bluetooth, to a second medical computing unit 102 and/or a third medical computing unit 103. In this way, a nurse using the second or third medical computing unit 102, 103 may have access to the treatment data of a certain patient when the nurse using the first medical computing unit 101 ends their shift. In some cases, the first medical computing unit 101 cannot reach the third medical computing unit 103 via the wireless protocol. Then, in certain embodiments, the second medical computing unit 102 may transmit the data to the third medical computing unit 103. This may be advantageous if the second computing 102 device is closer to the third medical computing unit 103 than the first medical computing unit 101 is .

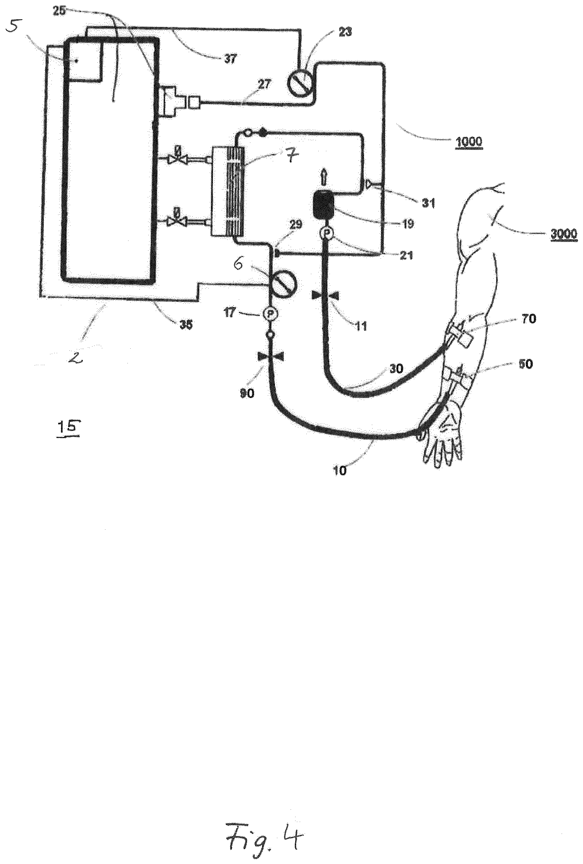

**Fig. 4** shows a medical device 1 according to the invention, which - as an example - is implemented as a blood treatment apparatus 15, in particular a dialysis machine 2.

In **Fig. 4****,** an extracorporeal blood circuit 1000 is connected to the dialysis machine 2 with a double needle access to the vascular system of a patient 3000. The extracorporeal blood circuit 1000 comprises an arterial line section 10 and a venous line section 30.

The arterial line section 10 is connected via an arterial patient access, for example, as shown in **Fig. 4****,** in the shape of an arterial connection needle 50, to the vascular system of the patient 3000. The venous line section 30 is connected via a venous patient access, for example, as shown in **Fig. 4****,** in the shape of a venous connection needle 70, to the vascular system of the patient 3000.

The arterial line section 10 comprises an arterial hose clamp 90, the venous line section 30 comprises a venous hose clamp 11 or may be connected therewith, respectively.

The extracorporeal blood circuit 1000 is inserted into a blood pump 6 of the dialysis machine 2. The blood pump 6 is implemented as a roller pump, for example. The blood pump 6 conveys the patient's 3000 blood through the extracorporeal blood circuit 1000.

The extracorporeal blood circuit 1000 comprises a blood treatment unit, e.g., a dialyzer 7, blood filter or the like.

As shown in **Fig. 4****,** an arterial pressure sensor 17 is arranged at the arterial line section 10 downstream (referring to the typical conveying direction during a blood treatment) of the blood pump 6.

In the venous line section 30, a venous drip chamber 19 and a venous pressure sensor 21 are contained or arranged upstream of the venous drip chamber 19.

Via a second, optional conveying unit, for example, a substituate pump 23, a substituate fluid may be introduced from a substituate fluid storage unit 25 via a substituate fluid feed line 27 to a predilution addition site 29 and/or a postdilution addition site 31 for substituate fluid into the extracorporeal blood circuit 1000. The substituate pump 23 is here, by way of example, implemented as a roller pump.

The blood treatment apparatus 15 comprises a control unit 5. The control unit 5 is connected to the blood pump 6 via a control line 35 and to the substituate pump 23 via a control line 37.

The control unit 5 may, for example, control a pump rate or a pump power of the blood pump 6 depending on operating parameters of the substituate pump 23, for example, its pump rate or pump power.

The control lines 35 and 37 may allow bidirectional communication between the control unit 5 and the blood pump 6 or the substituate pump 23, respectively.

**Fig. 4** shows a connection of the dialysis machine 2 to the vascular system of the patient 3000. Such a connection is not a part of the dialysis machine 2, but is merely a specific embodiment and a preferred use.

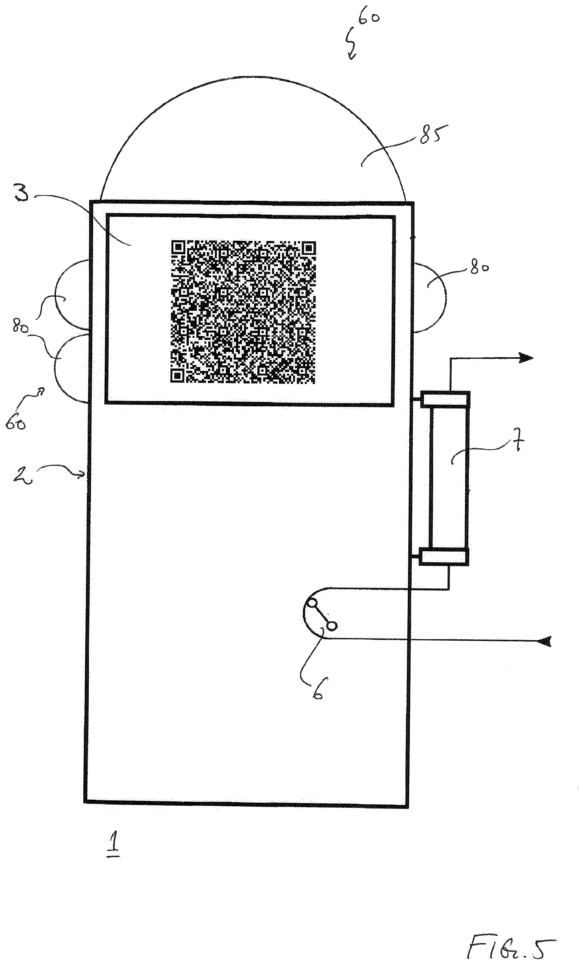

**Fig. 5** shows a medical device 1 according to the invention implemented as a dialysis machine 2 with a display 3 showing a QR code. The dialysis machine 2 further comprises a blood pump 6 and a dialyzer 7. To prevent electromagnetic interference and increase electromagnetic compatibility, the medical device 1 comprises a protective element 60, which reduces the amount of electromagnetic radiation emitted by the medical computing unit 100, which reaches the medical device 1. In **Fig. 5****,** the protective element 60 is implemented as a spacer 80, which sits on the surface of the medical device 1 and prevents medical computing units 100 from being placed too close. The spacer 80 may take any shape or form suitable to keep a medical computing unit 100 away from the surface of the medical device 1 while not impeding its function. Alternatively or additionally, the protective element 60 can be implemented as a protective surface 85, which may be slanted or rounded, such that a medical computing unit 100 may not be placed stably on top of the protective surface 85. In the example shown, the top surface of the medical device 1 is rounded. A medical computing unit 100 placed on top of the rounded protective 85 surface would likely slide down, such that a user would avoid placing the medical computing unit 100 on the protective surface 85.

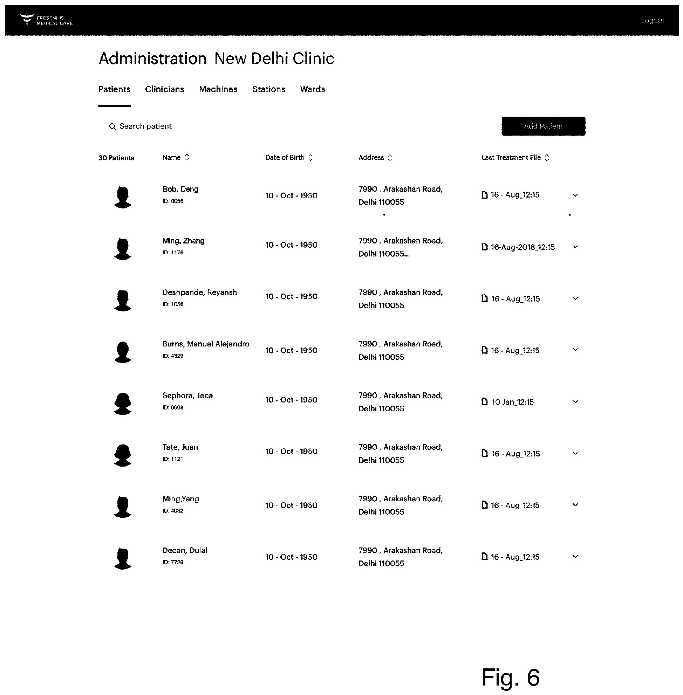

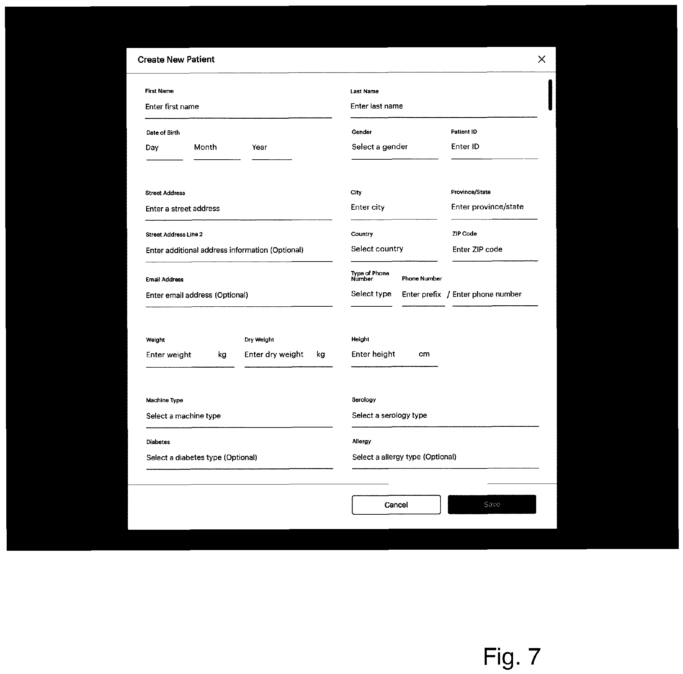

**Fig. 6** shows an administration screen on the display 3 of a medical computing unit 100 such as a tablet or a desktop PC. In the following, selecting an option on a screen is sometimes referred to as touching a certain field or button. This presumes the existence of a touchscreen on the medical computing unit 100. In alternative embodiments without a touchscreen, selection is carried out in a different way, e.g., using a mouse. A user such as a medical professional, in particular a nurse, is logged into the medical computing unit 100. On the top right-hand corner, a field allows to log the professional out of the medical computing unit 100 by selecting "Logout". The current screen shown in **Fig. 6** serves to administrate a dialysis clinic. At the top of the screen, under the headline "administration" and the name of the clinic, several options can be chosen. In the embodiment shown, the options are: "patients", "clinicians", "machines", "stations" and "wards". Currently, the option "patients" is chosen and, accordingly, a list of patients is shown. Each patient is represented with a symbol, which may be generic symbol only showing the gender of the patient, or a photo of the patient for ease of identification and avoidance of confusion and assignment errors. Furthermore, each patient is listed with their name and an ID number, which is a unique identifier and which, in the example shown, is a four-digit number. The date of birth and address are listed for every patient as well as the last treatment date on file. The user can add a new patient by pressing or clicking on the "add patient" button above the patient list on the right, which leads to a data entry mask being displayed, such as the one in **Fig. 7****.**

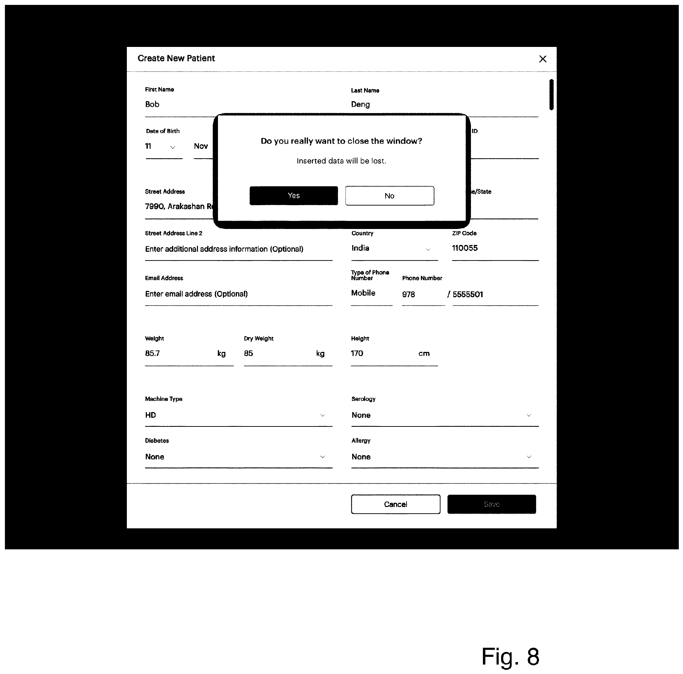

The mask in **Fig. 7** allows to enter a patient's details such as first and last name, date of birth, gender, patient ID, address (including street address, city, province, country and ZIP code), e-mail address, phone number, weight, dry weight, height, machine type, serology, diabetes type and potential allergies. Some of the details may be optional. For example, the type of diabetes may not have to be entered, as not all patients may be suffering from diabetes. The mask may be displayed as a pop-up window in front of the list in **Fig. 6****.** Alternatively, the mask may pop up while the background is hidden such as in **Fig. 7****.** After data entry, the user has the possibility to save patient data by clicking or touching the "save" button. Alternatively, the user may choose "cancel" to terminate data entry. In this case, a safety message may be displayed, such as the one in **Fig. 8** asking to confirm the termination of the addition of the new patient. The safety message may be contained in another pop-up window on top of the data entry mask and may clarify that any data concerning the new patient already entered will be lost if the data entry is terminated. If the user confirms that the data entry should be terminated, the data entry is stopped. Alternatively, the user may choose to go back to the data entry mask to save the data already entered or to enter further patient data. These two options are shown in **Fig. 8** in a pop-up window under the heading "do you really want to close the window" with the response options being "yes" and "no".

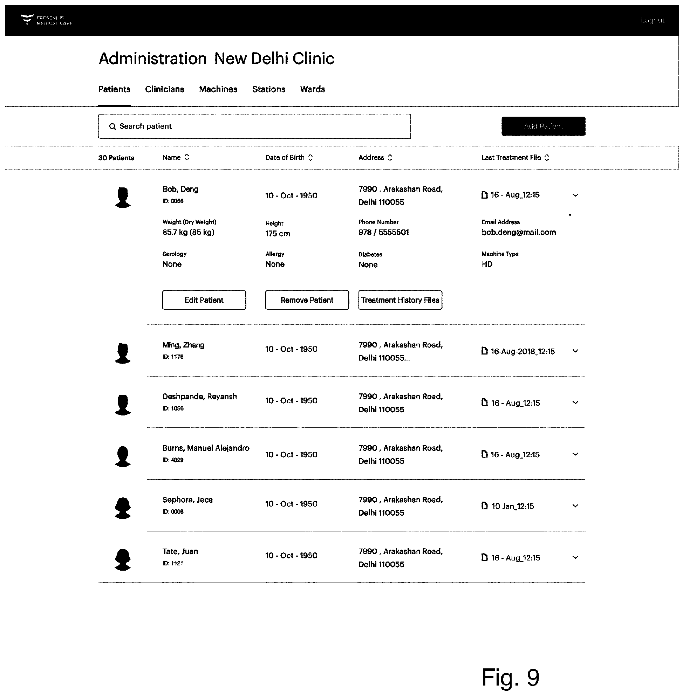

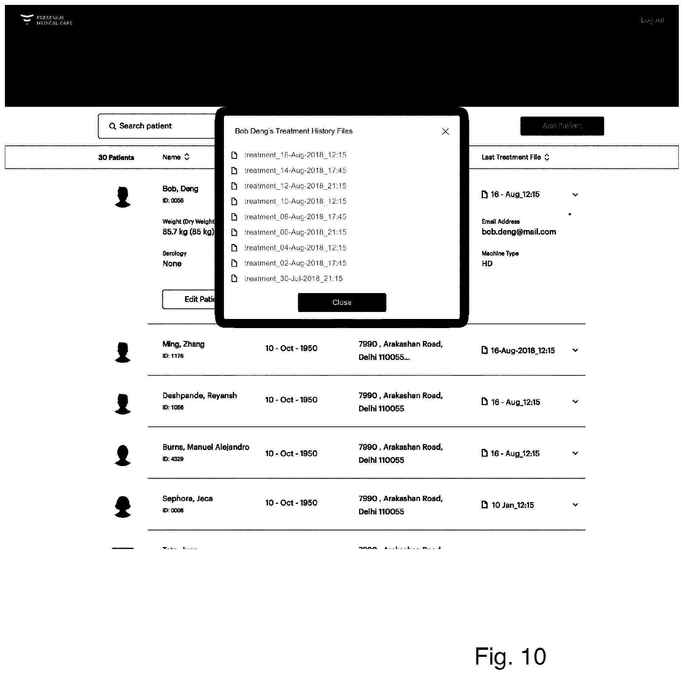

From the patient list shown in **Fig. 6****,** the user may select a patient by clicking or touching the patient's symbol or photo. In some embodiments, the patient may be selected by touching anywhere on the line, in which the patient's data is displayed. This may cause the line to expand vertically to display further patient data, such as further address details, e-mail address, machine type, diabetes type, allergies, weight, height and/or serology. In addition, further options concerning the selected patient are displayed. These options may include buttons for "edit patient", "remove patient" and/or "treatment history" as is shown in **Fig. 9****.** Selecting "edit patient" may lead to a data entry mask like the one shown in **Fig. 7****,** in which available patient data is displayed. The user may then change a specific data entry or add further data. Alternatively, the user may remove the patient from the list by selecting "remove patient". In some cases, this may delete all patient data from the current medical computing unit 100 and optionally from further medical computing units 100 and/or servers. Alternatively, the patient may just be deleted from the list whereas patient data may be retained in case the patient is added again at a later point in time. If the button "treatment history files" is selected, the treatment history data may be downloaded or shown. Once the "treatment history files" button is selected, a pop-up window such as the one shown in **Fig. 10** may appear. Inside the window, the patient's name may be displayed as well as the available treatment history files. The available files have different file names, e.g., including the treatment date and time. By selecting one of the files, the user may be able to display the treatment data created on a specific date. Alternatively, the user may be able to download the data for offline use.

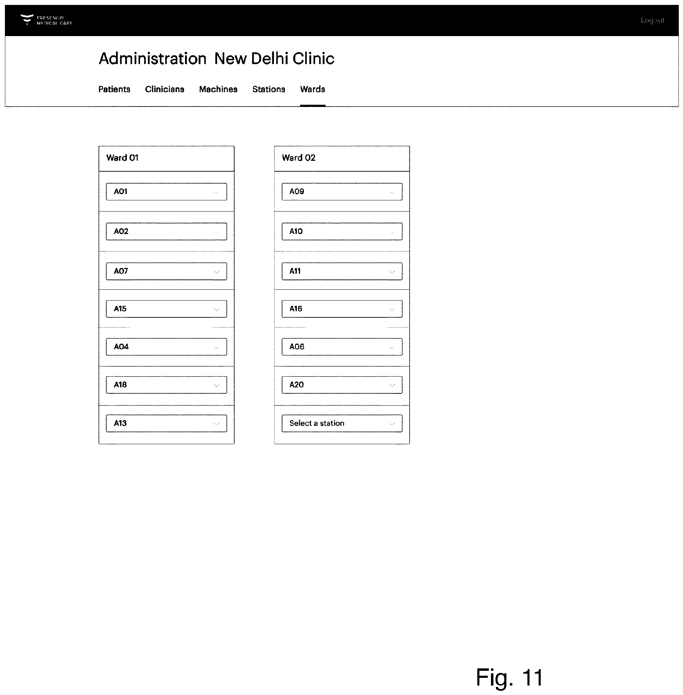

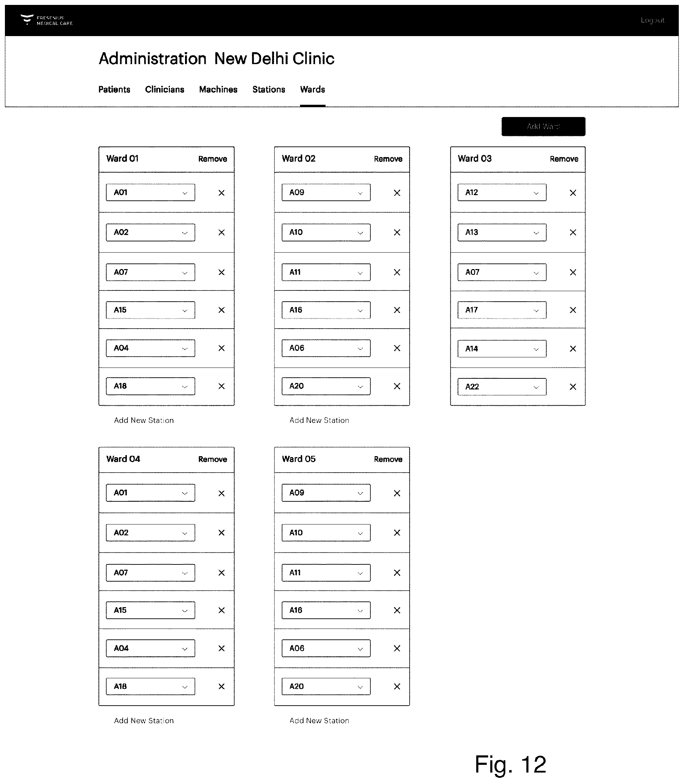

**Fig. 11** shows an administration screen similar to the one shown in **Fig. 6** with the "wards" option chosen. In the example shown, two boxes are displayed, each representing one ward. Each box shows the ward number on the top and a number of dialysis stations below, which are represented by identifiers (e.g., one letter and a two-digit number, such as A01). Further dialysis stations may be added to a specific ward. Dialysis stations may also be chosen or changed for each ward. If the user wishes more wards to be added to the screen, there may be an "add ward" button to do so as shown in **Fig. 12****.**

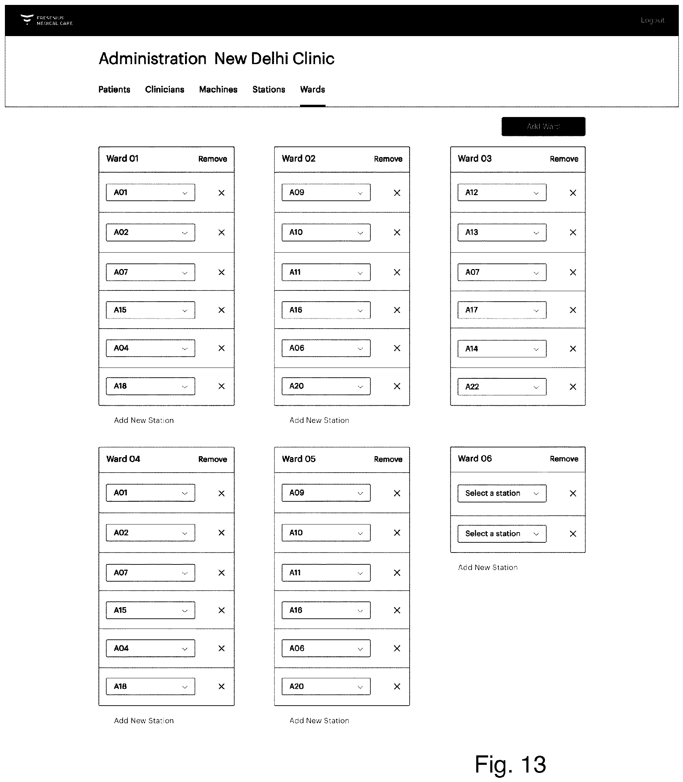

In **Fig. 12****,** five wards are shown in one box each. Each of the wards has, e.g., six dialysis stations. Dialysis stations may be removed from a ward or further stations may be added to a ward. Also, entire wards may be added or removed. In each box representing a ward there are lines representing a dialysis station. For each line, the identifier of a dialysis station may be chosen. In this way, a ward does not necessarily contain a contiguous set of dialysis machines 2. **Fig. 13** shows the addition of a sixth ward to the five wards already present. The new ward is shown as a box, which contains two lines with the words "select a station" in a drop-down list to be able to assign dialysis stations to the new ward.

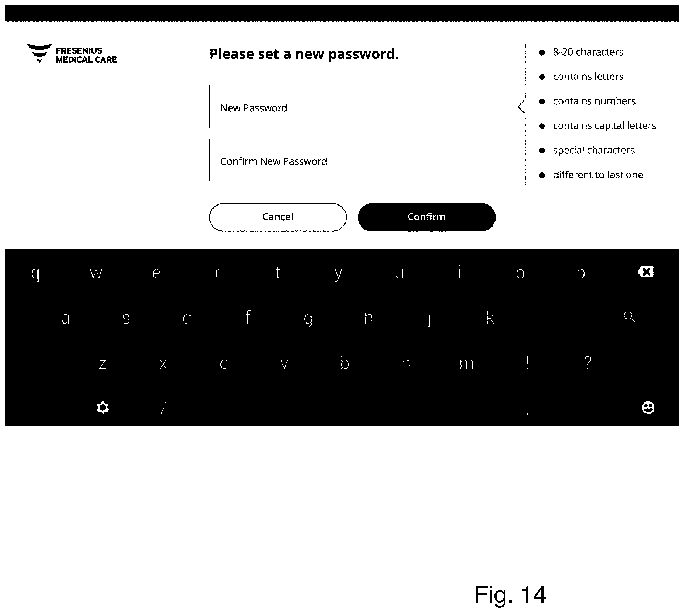

**Fig. 14** shows the screen of a medical computing unit 100, which is preferably a mobile device, such as a tablet. A user may log into the device using a username and a password. A password can preferably be set on the device itself. **Fig. 14** shows a screen used for setting a password with an on-screen keyboard to be displayed on a medical computing unit 100, such as a tablet with a touchscreen. The screen requires that the password be entered twice to change it, into a first and a second password field. In addition, the screen shows the requirements the new password must meet. After typing the password into the password fields, the user can press the "confirm" button to set the new password or press "cancel" to stop the process of setting a new password.

When a certain condition is met, the screen of the medical computing unit 100 may become locked. The condition that results in the screen becoming locked may be that the touch screen of the medical computing unit 100 has not been touched for a specific period of time, the medical computing unit 100 has not detected any movement for a specific period of time, the medical computing unit 100 has not been used for a specific period of time, the medical computing unit 100 is placed inside or outside a predefined area, the medical computing unit 100 is connected to a charger, the medical computing unit 100 has run out of battery and/or that the medical computing unit 100 loses Wi-Fi signal.

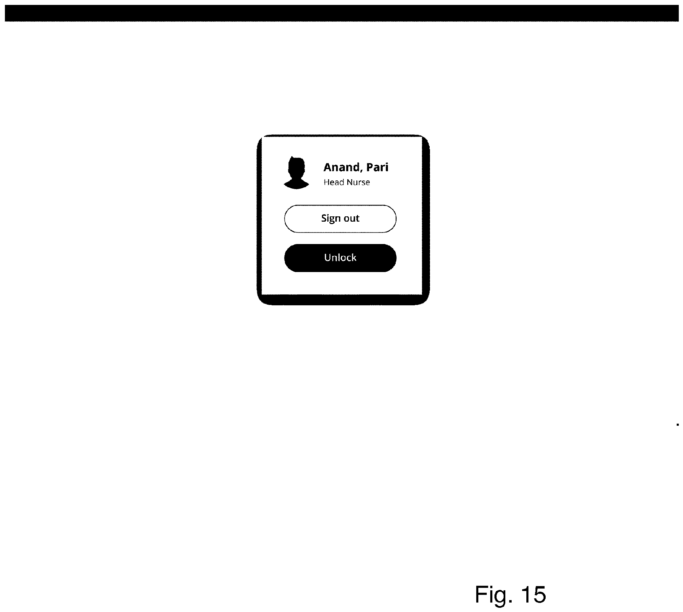

If the user tries to access the medical computing unit 100 while its screen is locked, a lock screen is displayed. An example of such a lock screen is shown in **Fig. 15****.** In the example shown, the lock screen shows the name, role and profile picture or symbol of the person currently logged in as well as buttons to sign out or unlock the medical computing unit 100. A user may unlock the medical computing unit 100, e.g., by entering a password, scanning a barcode or providing biometric identification.

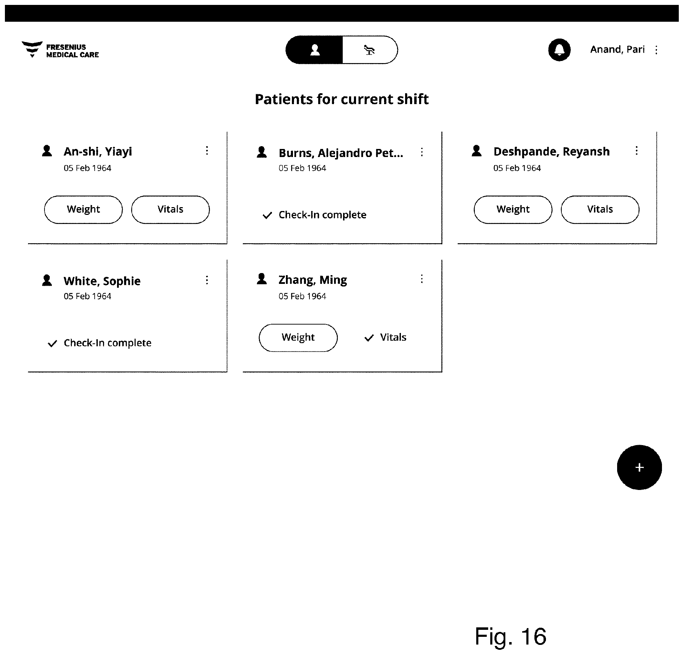

After unlocking the screen, the user may see a list or an array of patients to be treated in the current shift. **Fig. 16** shows an array of patient cards containing patient details (name, date of birth and profile picture or symbol). The name of the user currently logged in is displayed in the top right-hand corner. When a patient arrives at the clinic, the nurse may weigh the patient and collect vitals data, e.g., by measuring pulse and blood pressure. The screen shown in **Fig. 16** allows to enter weight and vitals data for each patient. Once such data has been entered, the check-in of the patient may be completed, which is shown on the screen in the patient card.

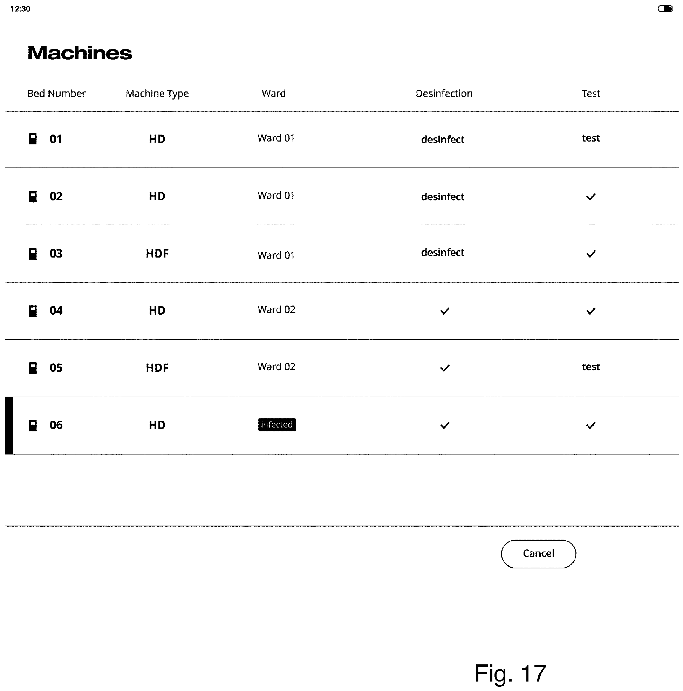

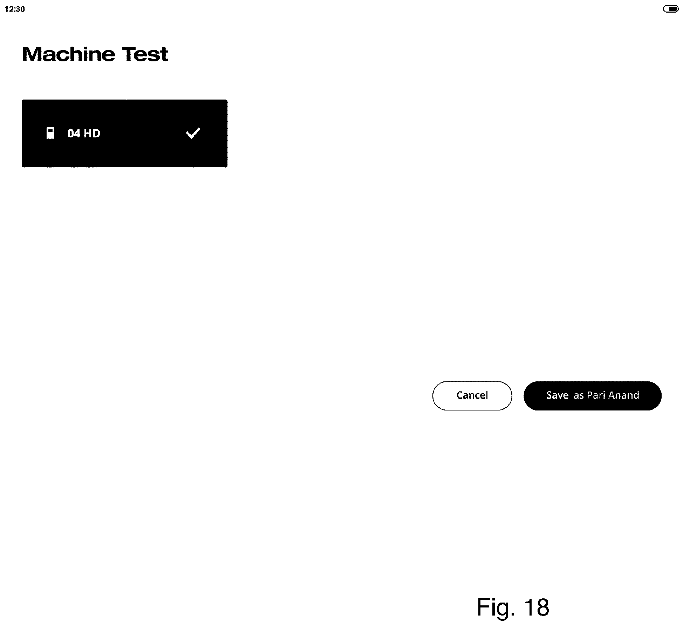

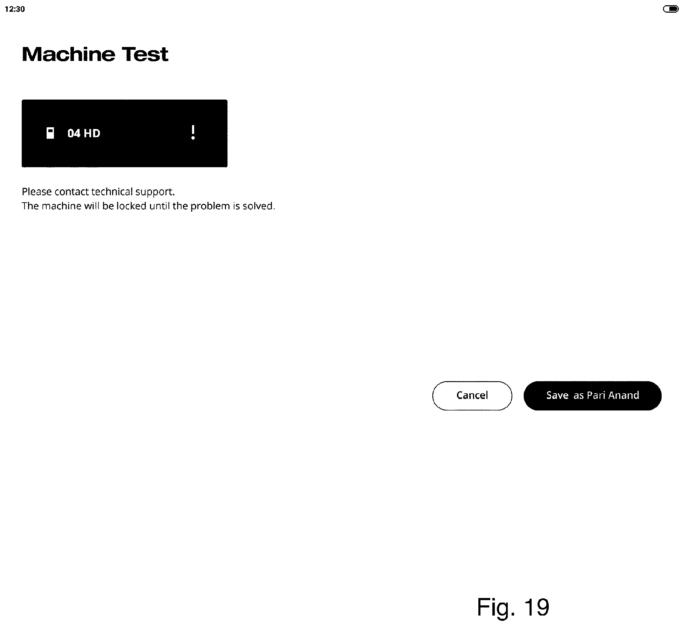

In **Fig. 17****,** the user can see an overview of dialysis machines 2. Each line specifies one machine, its type, bed number, ward, disinfection and test status. If a patient with an infectious disease has been treated at a dialysis machine 2, that dialysis machine 2 is listed as "infected" and may require special decontamination. One look enables the user to see which dialysis machines 2 are available to be used immediately. If the user selects one machine, a details screen such as the one in **Fig. 18** is shown. The checkmark in the box representing the dialysis machine 2 indicates that the machine is available. Furthermore, a color of the box, such as green, may additionally or alternatively indicate that the dialysis machine 2 is available. The screen allows assigning the selected dialysis machine 2 to the currently logged in user. If the dialysis machine 2 is not functioning correctly, the details screen may state a warning message and/or may prompt the user to contact technical support as shown in **Fig. 19****.** In this case, the box may have a different color, e.g., red and/or an exclamation mark may be displayed inside the box.

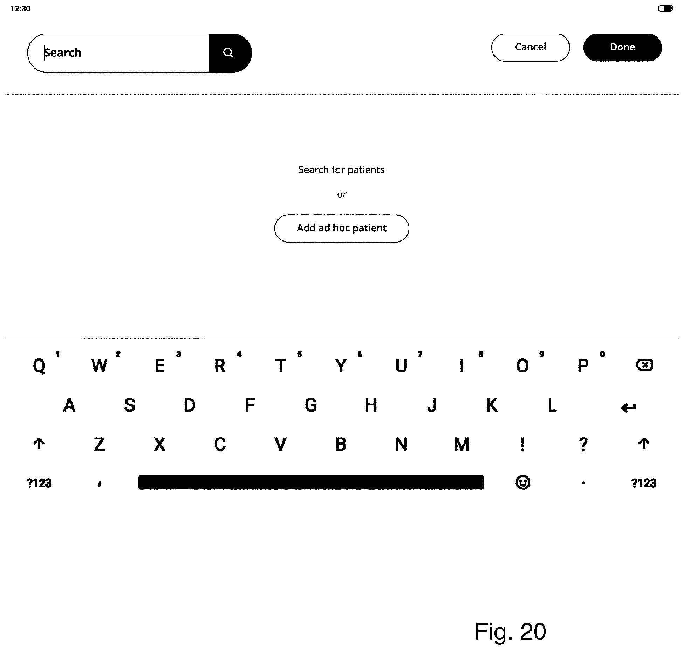

A patient may be chosen to be treated on the dialysis machine 2. If a specific patient is to be treated, the user may search for the patient by name. To this end, a search screen, such as the one in **Fig. 20****,** may be displayed on the medical computing unit 100. An on-screen keyboard allows entering a patient's details to search for a patient dataset or to create a new "ad hoc" patient on the medical computing unit 100.

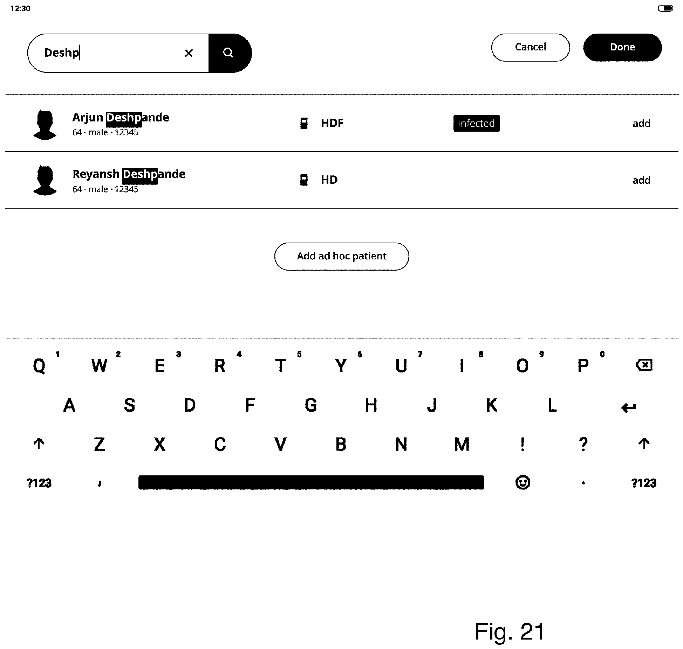

The medical computing unit 100 may carry out a live search with just a few letters of the patient's name entered into the search field. In the example of **Fig. 21****,** the user has entered "Deshp" into the search field, which yields two search results displayed immediately. Alternatively, the search may not be carried out live, but require for the search button to be pressed after entering search data. Each result found represents one patient. The search result may show patient name, ID number, age, gender, required dialysis machine type and infection status. Additionally, an "add" button may be displayed next to each patient found in the search. Such a button allows choosing the patient for treatment by the current user, e.g., during the current shift.

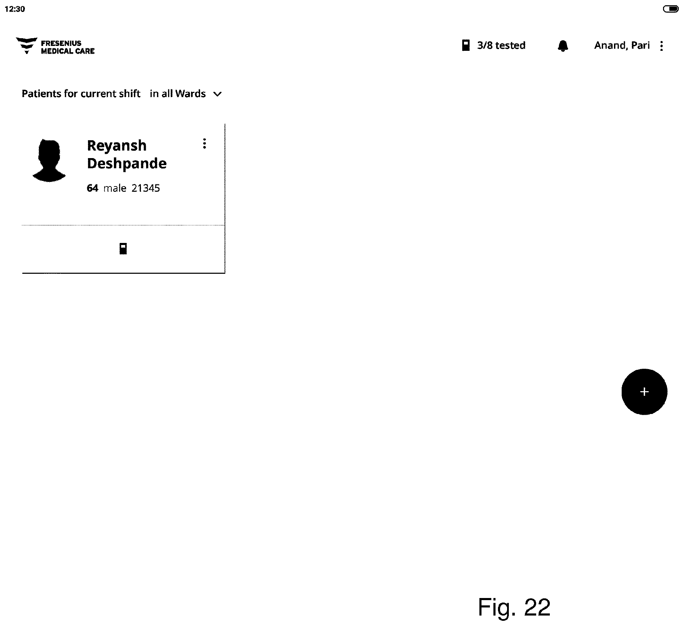

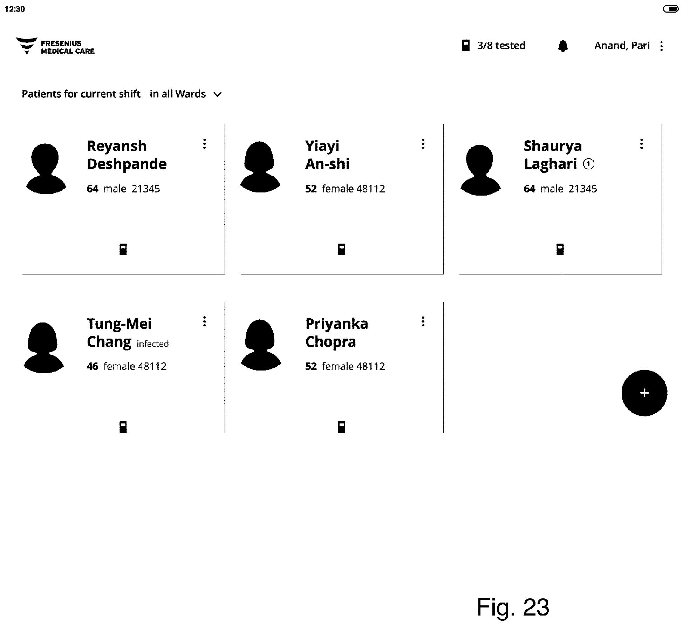

If the selection of the patient by the user is successful, a checkmark is displayed in the line with the patient's name. The user may display all the patients already assigned to the user for the current shift on a specific overview screen. Such a screen is shown in **Fig. 22** with just one patient. **Fig. 23** shows such a screen with five patients already assigned to the user. Each patient is represented by a card showing their profile picture or symbol, name, age, gender and ID number.

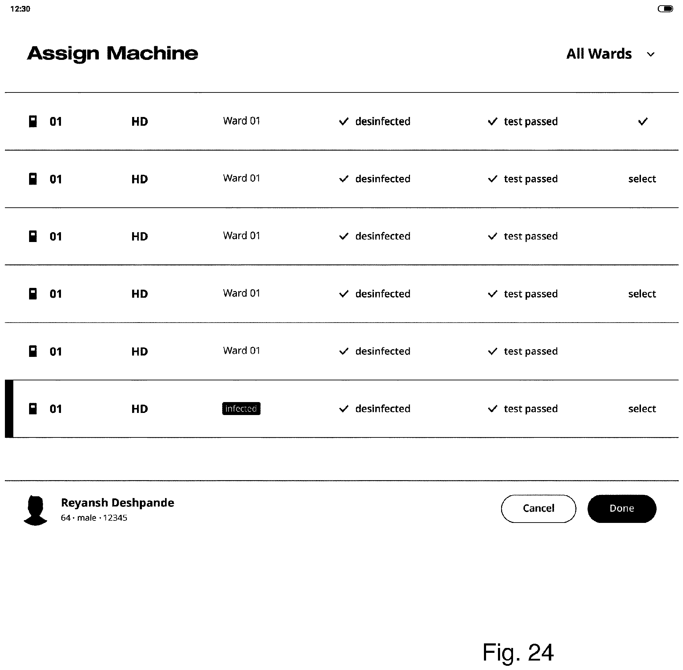

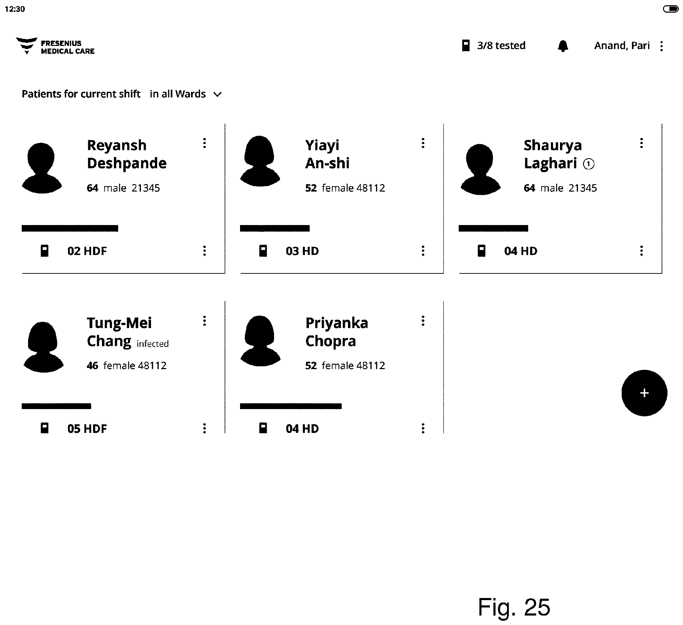

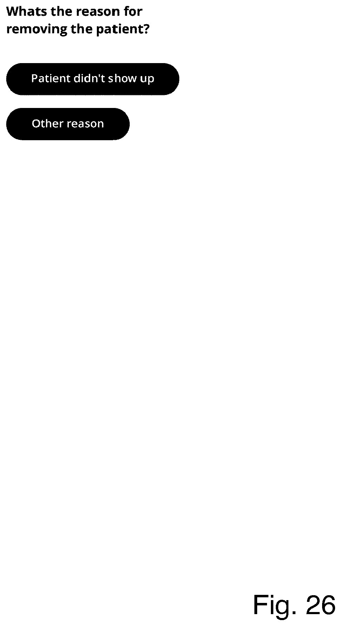

The user may choose one patient card from **Fig. 22** or **23****,** which will lead the medical computing unit 100 to display a list of dialysis machines 2 available, which is shown in **Fig. 24****.** If the user selects an available dialysis machine 2 and presses "done", then an assignment between patient and dialysis machine 2 is made. The overview screen will then show the patient card with the dialysis machine 2 assigned to the patient, such as shown in **Fig. 25****.** Additionally, the patient card may show a progress bar indicating how far the patient's treatment has progressed. If a patient is shown in the overview screen, but needs to be removed, each card may contain a menu activated by touching the three dots shown in **Fig. 25** next to the patient's name. After selecting the option to remove a patient, a window pops up asking for the reason of removal. Answering options may include "patient did not show up" and "other reason", such as shown in **Fig. 26****.**

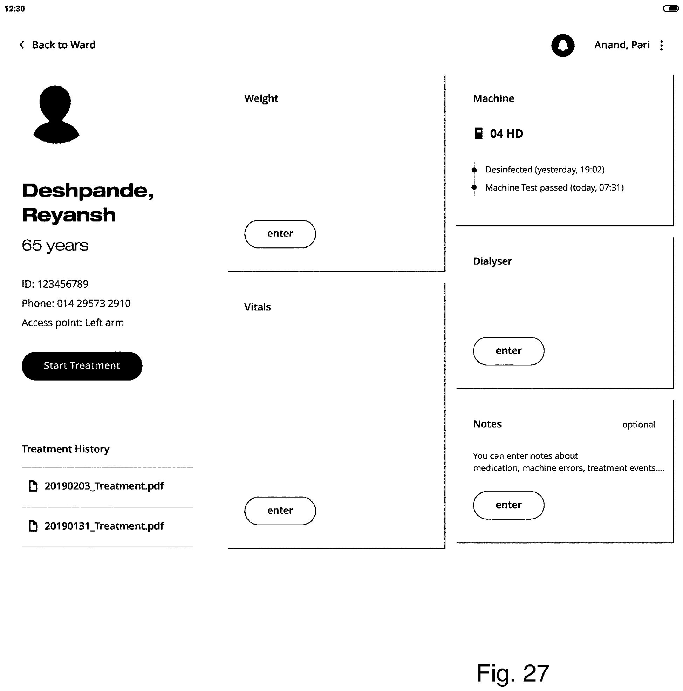

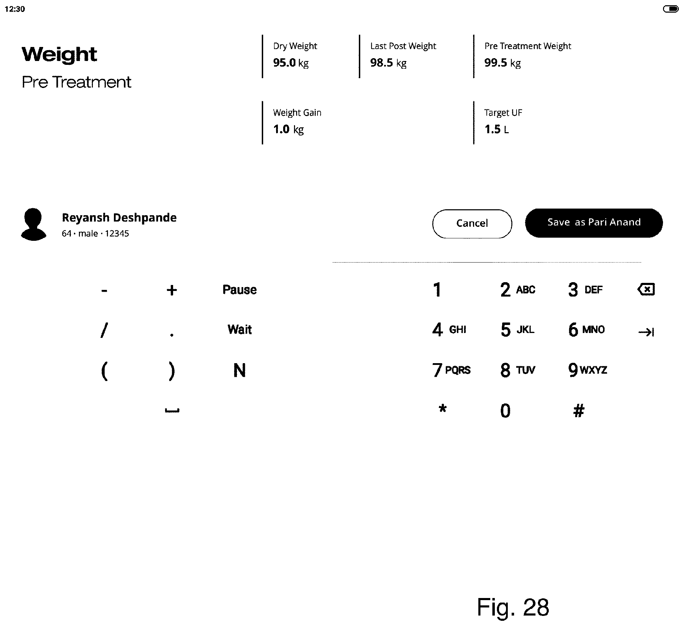

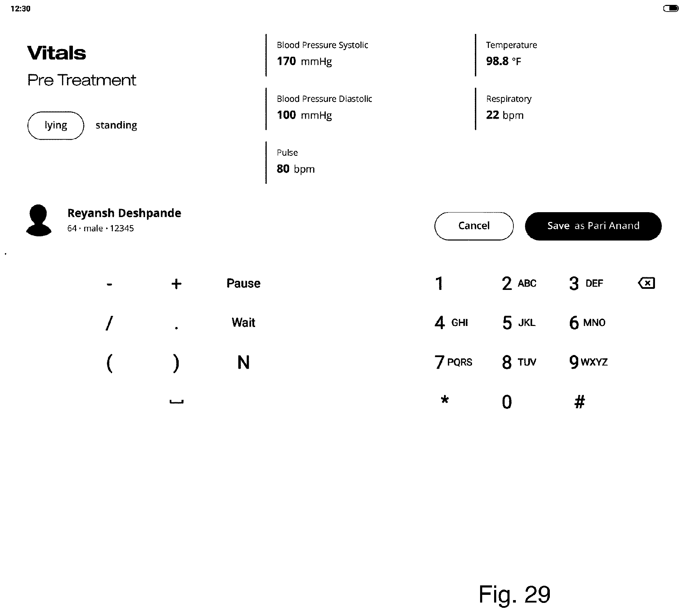

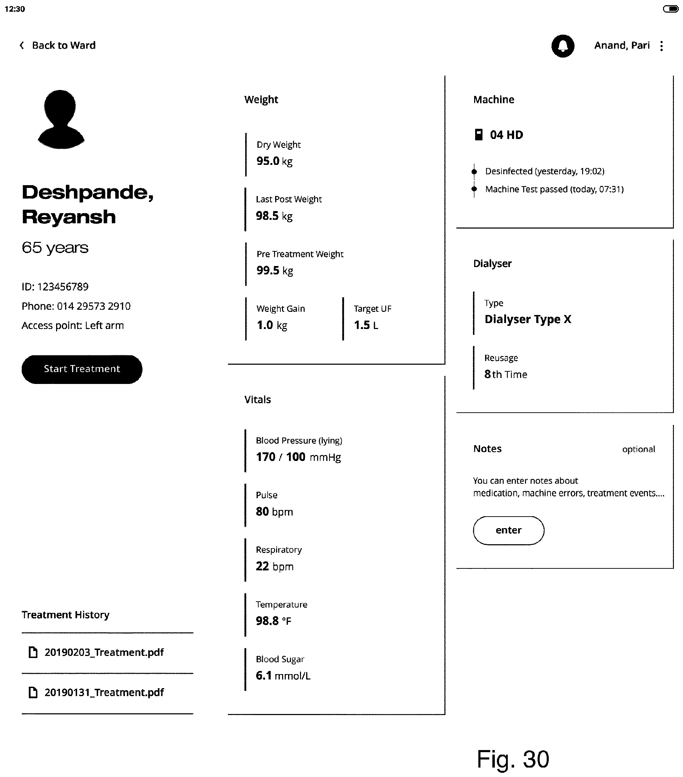

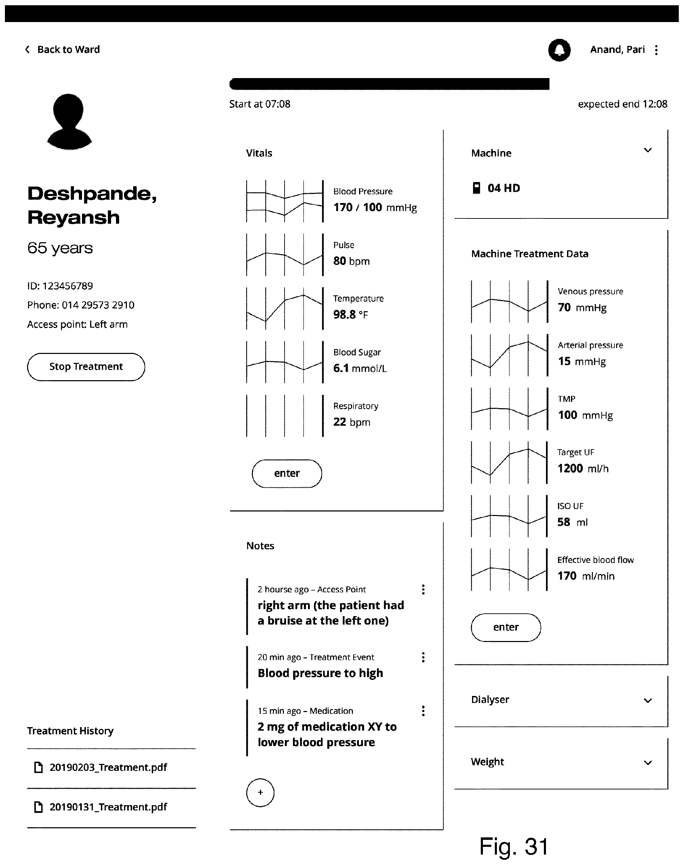

If the user wishes to start treatment, they will select the corresponding patient card from the overview screen. Subsequently, the patient's details are displayed full screen, such as is shown in **Fig. 27****.** The details shown may include the patient's name, age, ID number and phone number. Furthermore, medical data may be displayed, such as the access point for treatment (e.g., the patient's left arm). In addition to this, past treatment data may be selected and viewed from the screen shown in **Fig. 27****.** The screen further shows details of the dialysis machine 2 assigned to the patient. The user may enter notes, patient weight and vital signs as well as data concerning the dialyzer. If the user chooses to enter patient weight, a screen such as the one shown in **Fig. 28** may be displayed. The screen allows entering the current pre-treatment weight via an on-screen keyboard. Additionally, target ultrafiltration volume and/or desired dry weight may be entered according to a prescription. Moreover, the last post-dialysis weight may be viewed and the difference between the last post-dialysis weight and the current weight may be displayed as weight gain. **Fig. 29** shows a screen accessible from the screen in **Fig. 27** by selecting the button "vitals". On the screen in **Fig. 29****,** the user may enter vitals data, such as blood pressure, body temperature, respiratory frequency and pulse. If the data entry on the screens in **Fig. 28** **and** **29** is completed, a full screen representation of the patient with all the newly entered data may look as shown in **Fig. 30****.** The screen in **Fig. 30** further shows the type of dialyzer and how many times it has been used previously. Furthermore, a button is displayed, which allows the start of treatment. After pressing the button, the screen changes to the one presented in **Fig. 31****,** which shows the starting time of treatment, a progress bar and an expected end of treatment. Now, a button is shown, which allows the termination of the treatment. Further buttons allow entering treatment data and notes. In some embodiments, the user may transfer data from the dialysis machine to the medical computing unit 100 during treatment. This may occur manually, such as via a screen shown in **Fig. 29****.** Alternatively, there may be a wireless transfer of data from the dialysis machine 2 to the medical computing unit 100. For example, the dialysis machine 2 may display a barcode, such as a QR code, containing relevant data. The user may then take a picture of the barcode, which the medical computing unit 100 may decode and store the information contained. Certain patient parameters may be measured several times during treatment, such as blood pressure, pulse, body temperature, blood sugar and/or respiratory frequency. Furthermore, some machine parameters may be measured multiple times, such as venous pressure, arterial pressure, transmembrane pressure, target ultrafiltration volume, isolated ultrafiltration and/or effective blood flow. Each of these parameters may be displayed as data points in a graph versus time as can be seen on **Fig. 31****.** It may be that the data points displayed were not collected at fixed time intervals. Nevertheless, in the graphs the time intervals between data points may be shown as constant for ease of viewing. The user may then easily recognize trends in certain parameters. In a notes section on **Fig. 31****,** the user may view recent events reported by the dialysis machine and/or notes entered manually. In the case shown in **Fig. 31****,** the nurse has protocolled that the right arm was used for access even though the left arm is listed as a preferred access point, the reason being that the patient had a bruise on the left arm. Furthermore, the event "blood pressure too high" is shown in the notes section of **Fig. 31****.** This may be an automatic report by the dialysis machine, which may have been transferred to the medical computing unit 100 by a barcode scan. Alternatively, it may be a manual note entered by the nurse or doctor. Another event shown in the notes section in **Fig. 31** relates to medication given at a specified time.

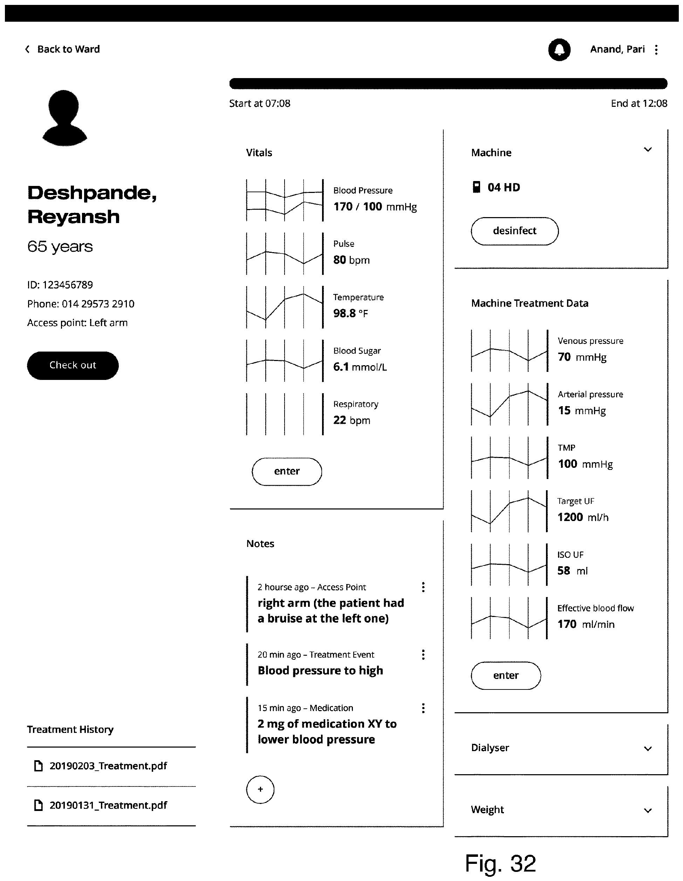

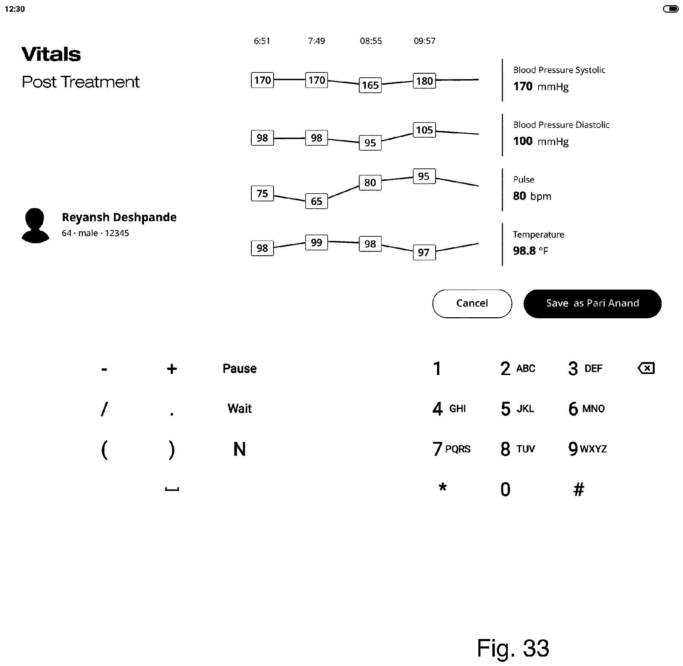

**Fig. 32** shows a screen, which is displayed when the treatment is completed. Below the progress bar, a start time and end time is shown. The complete progress bar being displayed indicates completion of the treatment. Next to the patient's details, a new button allows the user to perform a checkout of the patient. The weight section is minimized, but can be expanded again. The machine section now shows a button for the disinfection of the dialysis machine. Furthermore, vitals data may be corrected by touching "enter" in the vitals section, which may lead to the screen shown in **Fig. 33****.**

After the end of treatment, **Fig. 33** shows a graph of vitals versus time, in particular blood pressure, pulse and body temperature are shown at the top of the screen. Values may be corrected or added post-treatment using an on-screen keyboard.

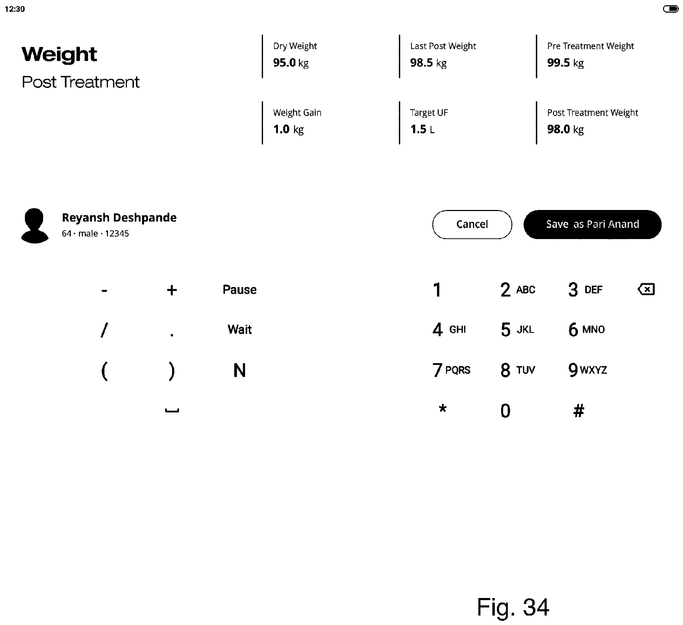

Similarly, the screen shown in **Fig. 34** allows manual correction and addition of weight values post-treatment. Once the patient has been weighed after the treatment, the post-treatment weight may be entered and saved. **Fig. 34** then shows a useful overview of the weight values, such as current post-treatment weight, previous post-treatment weight, current pre-treatment weight and/or weight gain between the end of the last treatment and the beginning of the current treatment. Furthermore, the target ultrafiltration volume is shown.

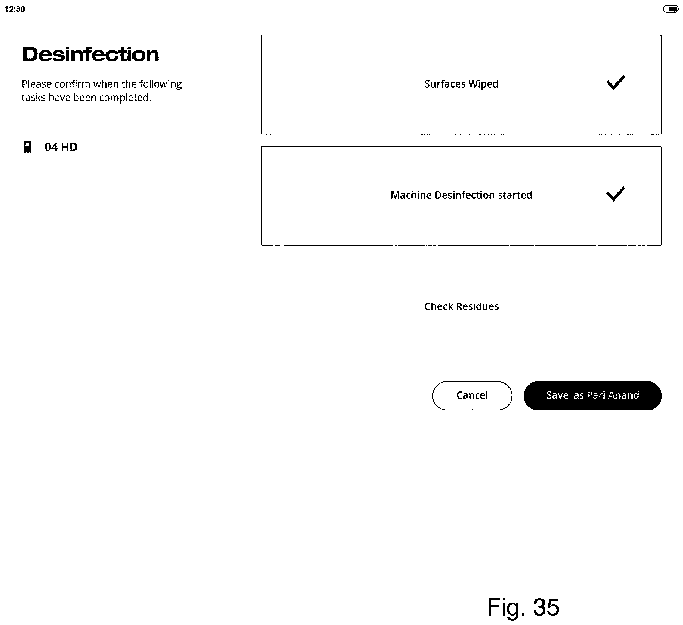

Post-treatment, the dialysis machine must be cleaned, which must be protocolled. Once the nurse has wiped the surfaces and has started the disinfection of the dialysis machine, they may confirm this on the disinfection page shown in **Fig. 35****.**

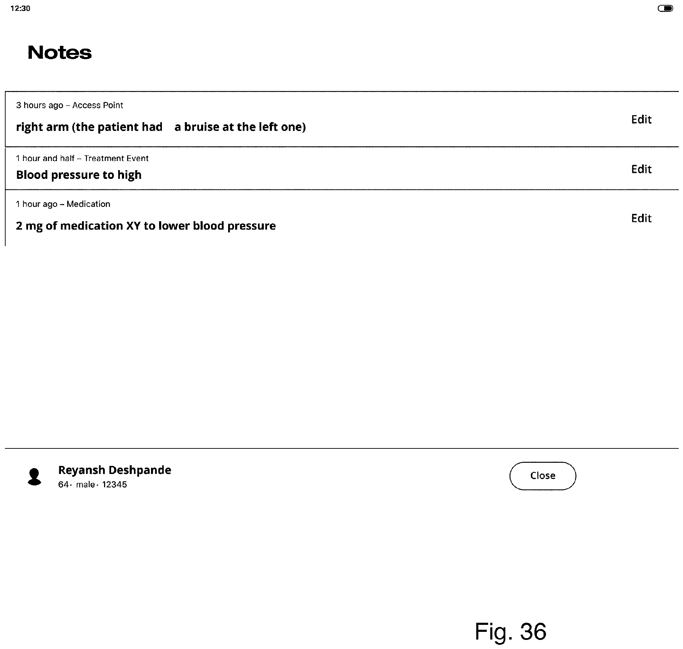

Furthermore, the user may review notes made during the treatment on a specific notes screen shown in **Fig. 36****.** Here, notes may be corrected or edited to include further details. The notes screen may also be useful to review the treatment. Some notes may not be editable due to legal requirements. For the same reasons, in some embodiments, the medical computing unit 100 may store the original note internally, even after it has been corrected.

### List of reference numerals

### Reference numeral list

- 1: medical device
- 2: dialysis machine
- 3: display
- 4: blood line set
- 5: control unit
- 6: blood pump
- 7: dialyzer
- 10: arterial line section
- 11: venous hose clamp
- 15: blood treatment apparatus
- 17: arterial pressure sensor
- 19: venous drip chamber
- 21: venous pressure sensor
- 23: substituate pump
- 25: substituate fluid storage unit
- 27: substituate fluid feed line
- 29: predilution addition site
- 30: venous line section
- 31: postdilution addition site
- 33: signal processing unit
- 35: control line to the blood pump
- 37: control line to the substituate pump
- 50: arterial connection needle
- 60: protective element
- 70: venous connection needle
- 80: spacer
- 85: protective surface
- 90: arterial hose clamp
- 100: medical computing unit
- 101: first medical computing unit
- 102: second medical computing unit
- 103: third medical computing unit
- 1000: extracorporeal blood circuit
- 3000: patient

## Claims

1. A method of deactivation of a medical computing unit (100) for processing patient data,
wherein the medical computing unit (100) is deactivated if a predefined condition is met.

2. The method according to claim 1, wherein the predefined condition is selected from at least one of the following:
- a touch screen of the medical computing unit (100) has not been touched for a specific period of time;
- an MPU, such as an accelerometer and/or magnetometer, of the medical computing unit (100) has not detected any movement of the medical computing unit (100) for a specific period of time;
- the medical computing unit (100) has not been used for a specific period of time;
- the medical computing unit (100) is placed inside or outside a predefined area;
- the medical computing unit (100) is connected to a charger;
- the medical computing unit (100) has run out of battery; and/or
- the medical computing unit (100) loses Wi-Fi signal.

3. The method according to claim 1 or 2, wherein the medical computing unit (100) is configured for reading of data from at least one medical device (1).

4. The method according to any one of the previous claims, wherein the reading is carried out optically.

5. The method according to claim 3 or 4, wherein the at least one medical device (1) is a blood treatment apparatus (15), in particular a dialysis machine (2).

6. A method of data synchronization comprising the following steps:
- a first medical computing unit (101) reads data from a medical device (1);
- the first medical computing unit (101) transmits the read data to a second medical computing unit (102).

7. The method according to claim 6, wherein the data is transmitted to a third medical computing unit (103) by the first and/or the second medical computing unit (101, 102) .

8. The method according to claim 6 or 7, wherein the data is displayed on the medical device (1) in a machine-readable graphical representation, such as in a 1D or 2D barcode or as a dynamic optical code, such as a flickercode.

9. A method of detecting proximity between a medical device (1) and a medical computing unit (100) with a wireless data transfer unit comprising the following step:
- detecting by the medical computing unit (100) and/or the medical device (1) if the medical computing unit (100) is located in close proximity to the medical device (1).

10. The method according to claim 9, wherein the proximity between the medical computing unit (100) and the medical device (1) is detected by a sensor of the medical computing unit (100) and/or the medical device (1).

11. The method according to claim 9 or 10, wherein when a close proximity between the medical computing unit (100) and the medical device (1) is detected, at least one of the following steps is carried out:
- reducing the power of the wireless data transfer unit of the medical computing unit (100);
- switching off the wireless data transfer unit of the medical computing unit (100); and/or
- playing a warning message on the medical computing unit (100) and/or the medical device (1).

12. A medical computing unit (100) configured to carry out the method of claim 1 to 11.

13. A medical device (1) configured to carry out the method of claim 3 to 11.

14. A medical device (1) with a protective element (60), which reduces the amount of electromagnetic radiation emitted by the medical computing unit (100), which reaches the medical device (1).

15. The medical device (1) according to claim 14, wherein the protective element (60) comprises a spacer (80), which is attached to the medical device (1).

16. The medical device (1) according to claim 14, wherein the protective element (60) is a protective surface (85) of the medical device (1) and/or the medical computing unit (100), which does not permit the stable placement of the medical computing unit (100) on the medical device (1).

17. A system of at least one medical computing unit (100) according to claim 12 and at least one medical device (1) according to claim 13 to 16.

18. A digital storage medium, in particular in the shape of a floppy disk, CD or DVD or EPROM, with electronically readable control signals, configured to interact with the medical device (1) and/or the medical computing unit (100), such that the machine implemented steps of the method according to one of the claims 1 to 11 are performed.

19. A computer program product with a program code stored on a machine readable carrier for executing the machine implemented steps of the method according to one of the claims 1 to 11, when the computer program product is running on the medical device (1) and/or the medical computing unit (100).

20. A computer program with a program code for prompting the machine implemented steps of the method according to one of the claims 1 to 11, when the computer program is executed on the medical device (1) and/or the medical computing unit (100).
